(19) Europäisches Patentamt
European Patent Office
Office européen des brevets

(11) EP 4 588 473 A1

(12) # EUROPEAN PATENT APPLICATION
published in accordance with Art. 153(4) EPC

(43) Date of publication:
**23.07.2025 Bulletin 2025/30**

(21) Application number: **23865452.9**

(22) Date of filing: **11.09.2023**

(51) International Patent Classification (IPC):
***A61K 31/16*** *(2006.01)* ***A61K 9/107*** *(2006.01)*
***A61K 9/14*** *(2006.01)* ***A61K 47/04*** *(2006.01)*
***A61K 47/10*** *(2017.01)* ***A61K 47/14*** *(2017.01)*
***A61K 47/32*** *(2006.01)* ***A61K 47/34*** *(2017.01)*
***A61K 47/36*** *(2006.01)* ***A61K 47/38*** *(2006.01)*

(52) Cooperative Patent Classification (CPC):
**A61K 9/107; A61K 9/14; A61K 31/16; A61K 47/02; A61K 47/10; A61K 47/14; A61K 47/32; A61K 47/34; A61K 47/36; A61K 47/38**

(86) International application number:
**PCT/JP2023/032990**

(87) International publication number:
**WO 2024/058099 (21.03.2024 Gazette 2024/12)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **12.09.2022 JP 2022144675**

(71) Applicant: **PTC Therapeutics, Inc.**
**Warren, NJ 07059 (US)**

(72) Inventors:
- **HOKONOHARA, Kazuhiro**
**Suita-shi, Osaka 564-0053 (JP)**
- **MARUYAMA, Megumi**
**Osaka-shi, Osaka 541-0045 (JP)**
- **HAN, Jin**
**Suita-shi, Osaka 564-0053 (JP)**
- **ONOUE, Yoshihiro**
**Suita-shi, Osaka 564-0053 (JP)**
- **NOBUTO, Tomohiro**
**Suita-shi, Osaka 564-0053 (JP)**

(74) Representative: **J A Kemp LLP**
**80 Turnmill Street**
**London EC1M 5QU (GB)**

(54) **GRANULES FOR SUSPENSION**

(57) Provided is a granule for suspension, which is a pharmaceutical composition including: a compound represented by formula 1:

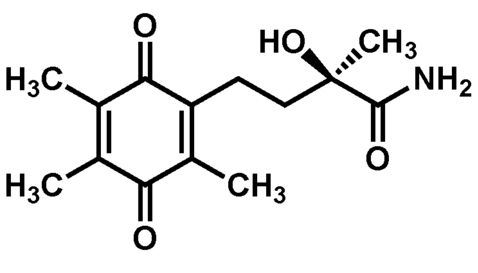

or a pharmaceutically acceptable salt or solvate thereof; a binding agent; and a dispersant. The binding agent includes a binding agent selected from the group consisting of a cellulose binding agent, a povidone binding agent, a vinyl alcohol binding agent, and a thickening polysaccharide binding agent. The dispersant is selected from the group consisting of an inorganic salt dispersant, an ether dispersant, a starch dispersant, a cellulose dispersant, and a fatty acid ester salt dispersant.

EP 4 588 473 A1

**Description**

BACKGROUND OF THE INVENTION

FIELD OF THE INVENTION

The present disclosure provides a pharmaceutical composition. The pharmaceutical composition can include a granule for suspension.

DISCUSSION OF THE BACKGROUND

International Publication No. WO 2009/061744 discloses a method of treating or suppressing mitochondrial diseases such as Friedreich's ataxia (FRDA), Leber hereditary optic neuropathy (LHON), mitochondrial myopathy, encephalopathy, lactic acidosis, and stroke (MELAS), Kearns-Sayre syndrome (KSS), and other disorders such as amyotrophic lateral sclerosis (ALS), Huntington's disease, Parkinson's disease, and pervasive developmental disorders such as autism, and describes compounds that are useful in said method, such as a 4-(p-quinolyl)-2-hydroxybutanamide derivative. The document also describes that that racemic 2-hydroxy-2-methyl-4-(2,4,5-trimethyl-3,6-dioxocyclohexa-1,4-dienyl)butanamide is synthesized from racemic Trolox (6-hydroxy-2,5,7,8-tetramethylchroman-2-carboxylic acid).

International Publication No. WO 2016/100579 describes polymorphic and amorphous forms of anhydrate, hydrate, and solvates of (R)-2-hydroxy-2-methyl-4-(2,4,5-trimethyl-3,6-dioxocyclohexa-1,4-dienyl)butanamide and methods of using such compositions for treating or suppressing oxidative stress disorders, including mitochondrial disorders, impaired energy processing disorders, neurodegenerative diseases and diseases of aging, as well as methods of making such polymorphic and amorphous forms.

International Publication No. WO 2021/167095 describes an optically resolved Trolox intermediate and a method for producing same.

SUMMARY OF THE INVENTION

As a result of diligent studies, the inventors discovered a granular agent for suspension with excellent properties of dispersibility and suppression of hydrate generation and crystal growth, including a compound represented by formula 1 or a pharmaceutically acceptable salt or solvate thereof.

For example, the present disclosure provides the following items.

(Item 1) A pharmaceutical composition comprising:

a compound represented by formula 1:

[Chemical Formula 2]

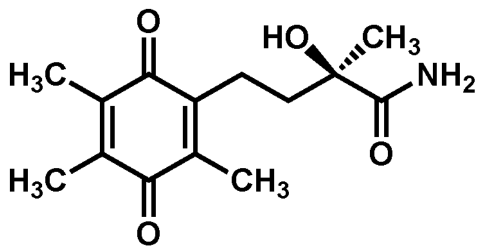

or a pharmaceutically acceptable salt or solvate thereof;
a binding agent; and
a dispersant.

(Item 2) The pharmaceutical composition of item 1, wherein the binding agent comprises a binding agent selected from the group consisting of a cellulose binding agent, a povidone binding agent, a vinyl alcohol binding agent, and a thickening polysaccharide binding agent.

(Item 3) The pharmaceutical composition of item 2, wherein the cellulose binding agent comprises a binding agent selected from the group consisting of hydroxypropyl cellulose, hypromellose, and methyl cellulose.

(Item 4) The pharmaceutical composition of item 2, wherein the povidone binding agent comprises a binding agent selected from the group consisting of povidone and copovidone.
(Item 5) The pharmaceutical composition of item 2, wherein the vinyl alcohol binding agent comprises a binding agent selected from the group consisting of polyvinyl alcohol and a polyvinyl alcohol-polyethylene glycol graft copolymer.
(Item 6) The pharmaceutical composition of item 2, wherein the thickening polysaccharide binding agent comprises xanthan gum.
(Item 7) The pharmaceutical composition of any one of items 1 to 6, wherein the dispersant is selected from the group consisting of an inorganic salt dispersant, an ether dispersant, a starch dispersant, a cellulose dispersant, and a fatty acid ester salt dispersant.
(Item 8) The pharmaceutical composition of item 7, wherein the inorganic salt dispersant comprises a dispersant selected from the group consisting of light anhydrous silicic acid and magnesium aluminometasilicate.
(Item 9) The pharmaceutical composition of item 7, wherein the ether dispersant comprises polyethylene glycol 6000.
(Item 10) The pharmaceutical composition of item 7, wherein the starch dispersant comprises corn starch.
(Item 11) The pharmaceutical composition of item 7, wherein the cellulose dispersant comprises a dispersant selected from the group consisting of crystalline cellulose/carmellose sodium, carmellose, and carmellose calcium.
(Item 12) The pharmaceutical composition of item 7, wherein the fatty acid ester salt dispersant comprises sodium stearyl fumarate.
(Item 13) The pharmaceutical composition of item 1, wherein

the binding agent comprises a cellulose binding agent, and
the dispersant comprises a dispersant selected from the group consisting of an inorganic salt dispersant, an ether dispersant, a starch dispersant, a cellulose dispersant, and a fatty acid ester salt dispersant.

(Item 14) The pharmaceutical composition of item 1, wherein

the binding agent comprises a cellulose binding agent, and
the dispersant comprises a dispersant selected from the group consisting of an inorganic salt dispersant, an ether dispersant, a starch dispersant, and a cellulose dispersant.

(Item 15) The pharmaceutical composition of item 1, wherein

the binding agent comprises a binding agent selected from the group consisting of hydroxypropyl cellulose, hypromellose, and methyl cellulose, and
the dispersant comprises a dispersant selected from the group consisting of light anhydrous silicic acid, polyethylene glycol 6000, corn starch, carmellose, crystalline cellulose/carmellose sodium, magnesium aluminometasilicate, sodium stearyl fumarate, and carmellose calcium.

(Item 16) The pharmaceutical composition of item 1, wherein

the binding agent comprises a binding agent selected from the group consisting of hydroxypropyl cellulose, hypromellose, and methyl cellulose, and
the dispersant comprises a dispersant selected from the group consisting of light anhydrous silicic acid, polyethylene glycol 6000, corn starch, and carmellose.

(Item 17) The pharmaceutical composition of item 1, wherein

the binding agent comprises hydroxypropyl cellulose, and
the dispersant comprises a dispersant selected from the group consisting of light anhydrous silicic acid, corn starch, carmellose, polyethylene glycol 6000, crystalline cellulose/carmellose sodium, magnesium aluminometasilicate, sodium stearyl fumarate, and carmellose calcium.

(Item 18) The pharmaceutical composition of item 1, wherein

the binding agent comprises hydroxypropyl cellulose, and
the dispersant comprises a dispersant selected from the group consisting of light anhydrous silicic acid, corn starch, and carmellose.

(Item 19) The pharmaceutical composition of item 1, wherein

the binding agent comprises hydroxypropyl cellulose, and
the dispersant comprises light anhydrous silicic acid.

(Item 20) The pharmaceutical composition of item 1, wherein

the binding agent comprises hypromellose, and
the dispersant comprises a dispersant selected from the group consisting of light anhydrous silicic acid and polyethylene glycol 6000.

(Item 21) The pharmaceutical composition of item 1, wherein

the binding agent comprises hypromellose, and
the dispersant comprises light anhydrous silicic acid.

(Item 22) The pharmaceutical composition of item 1, wherein

the binding agent comprises methyl cellulose, and
the dispersant comprises light anhydrous silicic acid.

(Item 23) The pharmaceutical composition of item 1, wherein

the binding agent comprises a povidone binding agent, and
the dispersant comprises a dispersant selected from the group consisting of an inorganic salt dispersant, an ether dispersant, a starch dispersant, a cellulose dispersant, and a fatty acid ester salt dispersant.

(Item 24) The pharmaceutical composition of item 1, wherein

the binding agent comprises a povidone binding agent, and
the dispersant comprises a dispersant selected from the group consisting of an inorganic salt dispersant and a cellulose dispersant.

(Item 25) The pharmaceutical composition of item 1, wherein

the binding agent comprises copovidone, and
the dispersant comprises a dispersant selected from the group consisting of light anhydrous silicic acid and crystalline cellulose/carmellose sodium.

(Item 26) The pharmaceutical composition of item 1, wherein

the binding agent comprises a cellulose binding agent, and
the dispersant comprises an inorganic salt dispersant.

(Item 27) The pharmaceutical composition of item 26, wherein

the binding agent comprises a binding agent selected from the group consisting of hydroxypropyl cellulose, hypromellose, and methyl cellulose, and
the dispersant comprises a dispersant selected from the group consisting of light anhydrous silicic acid and magnesium aluminometasilicate.

(Item 28) The pharmaceutical composition of item 27, wherein

the binding agent comprises a binding agent selected from the group consisting of hydroxypropyl cellulose, hypromellose, and methyl cellulose, and
the dispersant comprises light anhydrous silicic acid.

(Item 29) The pharmaceutical composition of item 28, wherein

the binding agent comprises a binding agent selected from the group consisting of hydroxypropyl cellulose and

hypromellose, and
the dispersant comprises light anhydrous silicic acid.

(Item 30) The pharmaceutical composition of item 1, wherein

the binding agent comprises a cellulose binding agent, and
the dispersant comprises an ether dispersant.

(Item 31) The pharmaceutical composition of item 30, wherein

the binding agent comprises a binding agent selected from the group consisting of hydroxypropyl cellulose and hypromellose, and
the dispersant comprises polyethylene glycol 6000.

(Item 32) The pharmaceutical composition of item 31, wherein

the binding agent compriseshypromellose, and
the dispersant comprises polyethylene glycol 6000.

(Item 33) The pharmaceutical composition of item 1, wherein

the binding agent comprises a cellulose binding agent, and
the dispersant comprises a starch dispersant.

(Item 34) The pharmaceutical composition of item 33, wherein

the binding agent comprises hydroxypropyl cellulose, and
the dispersant comprises corn starch.

(Item 35) The pharmaceutical composition of item 1, wherein

the binding agent comprises a cellulose binding agent, and
the dispersant comprises a cellulose dispersant.

(Item 36) The pharmaceutical composition of item 35, wherein

the binding agent comprises hydroxypropyl cellulose, and
the dispersant comprises a dispersant selected from carmellose, crystalline cellulose/carmellose sodium, and carmellose calcium.

(Item 37) The pharmaceutical composition of item 36, wherein

the binding agent comprises hydroxypropyl cellulose, and
the dispersant comprises a dispersant selected from crystalline cellulose/carmellose sodium and carmellose calcium.

(Item 38) The pharmaceutical composition of item 36, wherein

the binding agent comprises hydroxypropyl cellulose, and
the dispersant comprises carmellose.

(Item 39) The pharmaceutical composition of item 1, wherein

the binding agent comprises a cellulose binding agent, and
the dispersant comprises a fatty acid ester salt dispersant.

(Item 40) The pharmaceutical composition of item 39, wherein

the binding agent comprises hydroxypropyl cellulose, and
the dispersant comprises sodium stearyl fumarate.

(Item 41) The pharmaceutical composition of item 1, wherein

the binding agent comprises a povidone binding agent, and
the dispersant comprises a cellulose dispersant.

(Item 42) The pharmaceutical composition of item 41, wherein

the binding agent comprises copovidone, and
the dispersant comprises crystalline cellulose/carmellose sodium.

(Item 43) The pharmaceutical composition of item 1, wherein

the binding agent comprises a povidone binding agent, and
the dispersant comprises an inorganic salt dispersant.

(Item 44) The pharmaceutical composition of item 43, wherein

the binding agent comprises a binding agent selected from the group consisting of copovidone, povidone (nominal K value: 29 to 32), and povidone (nominal K value: 17), and
the dispersant comprises light anhydrous silicic acid.

(Item 45) The pharmaceutical composition of item 44, wherein

the binding agent comprises a binding agent selected from the group consisting of copovidone and povidone (nominal K value: 29 to 32), and
the dispersant comprises light anhydrous silicic acid.

(Item 46) The pharmaceutical composition of item 1, wherein

the binding agent comprises a vinyl alcohol binding agent, and
the dispersant comprises an inorganic salt dispersant.

(Item 47) The pharmaceutical composition of item 46, wherein

the binding agent comprises a binding agent selected from the group consisting of polyvinyl alcohol and a polyvinyl alcohol/polyethylene glycol/graft copolymer, and
the dispersant comprises light anhydrous silicic acid.

(Item 48) The pharmaceutical composition of item 47, wherein

the binding agent comprises a polyvinyl alcohol/polyethylene glycol/graft copolymer, and
the dispersant comprises light anhydrous silicic acid.

(Item 49) The pharmaceutical composition of item 1, wherein

the binding agent comprises a vinyl alcohol binding agent, and
the dispersant comprises a cellulose dispersant.

(Item 50) The pharmaceutical composition of item 49, wherein

the binding agent comprises a binding agent selected from the group consisting of polyvinyl alcohol and a polyvinyl alcohol/polyethylene glycol/graft copolymer, and
the dispersant comprises crystalline cellulose/carmellose sodium.

(Item 51) The pharmaceutical composition of item 1, wherein

the binding agent comprises a thickening polysaccharide binding agent, and
the dispersant comprises an inorganic salt dispersant.

(Item 52) The pharmaceutical composition of item 51, wherein

the binding agent comprises xanthan gum, and
the dispersant comprises light anhydrous silicic acid.

(Item 53) The pharmaceutical composition of any one of items 1 to 52, wherein the pharmaceutical composition comprises a suspending agent.
(Item 54) The pharmaceutical composition of item 53, wherein the suspending agent comprises a carboxyvinyl polymer.
(Item 55) The pharmaceutical composition of any one of items 1 to 54, wherein the pharmaceutical composition comprises a sweetener.
(Item 56) The pharmaceutical composition of item 55, wherein the sweetener comprises sucralose.
(Item 57) The pharmaceutical composition of any one of items 1 to 56, wherein the pharmaceutical composition comprises 1 mg to 1000 mg of binding agent with respect to 1000 mg of a compound represented by formula I or a pharmaceutically acceptable salt or solvate thereof.
(Item 58) The pharmaceutical composition of any one of items 1 to 57, wherein the pharmaceutical composition comprises a dispersant at 0.01 to 10% by weight with respect to the weight of the entire composition.
(Item 59) The pharmaceutical composition of any one of items 1 to 58, wherein the pharmaceutical composition comprises 1 mg to 1000 mg of suspending agent with respect to 1000 mg of a compound represented by formula I or a pharmaceutically acceptable salt or solvate thereof.
(Item 60) The pharmaceutical composition of any one of items 1 to 59, wherein the pharmaceutical composition comprises 1 mg to 1000 mg of sweetener with respect to 1000 mg of a compound represented by formula I or a pharmaceutically acceptable salt or solvate thereof.
(Item 61) A granule for suspension for the manufacture of a pharmaceutical composition, comprising the pharmaceutical composition of any one of items 1 to 60.
(Item 62) The granule for suspension of item 61, which is a granule for suspension for the preparation of a suspension.
(Item 63) A granule for suspension for the manufacture of a pharmaceutical composition, wherein the granule for suspension is the pharmaceutical composition of any one of items 1 to 60.
(Item 64) The granule for suspension of item 63, which is a granule for suspension for the preparation of a suspension.
(Item 65) The pharmaceutical composition of any one of items 1 to 60, which is a granule for suspension for the manufacture of a pharmaceutical composition.
(Item 66) The pharmaceutical composition of item 65, which is a granule for suspension for the preparation of a suspension.
(Item 67) A suspension comprising the pharmaceutical composition of any one of items 1 to 60, which is a granule for suspension for the manufacture of a pharmaceutical composition.
(Item 68) The pharmaceutical composition of any one of items 1 to 60, which is in a suspended state.
(Item 69) A method of treating or preventing a disease in a subject in need thereof, comprising administering to the subject in need thereof an effective amount of a pharmaceutical composition of any one of items 1 to 60, 65 to 66 or 68 or a suspension of item 67.
(Item 70) A method for producing a pharmaceutical composition of any one of items 1 to 60, 65 to 66 or 68 or a suspension of item 67, comprising mixing said compound or a pharmaceutically acceptable salt or solvate thereof, said binding agent and a dispersant.
(Item 71) A method for producing a pharmaceutical composition, comprising mixing the granules for suspension of any one of items 61 or 62 with necessary additives and/or suspension solvents (e.g., including water solvents such as purified water).
(Item 72) A method of preparing a pharmaceutical composition for administration to a subject in a medical setting, comprising mixing the granules for suspension of any one of items 63 or 64 with necessary additives and/or suspension solvents (e.g., including water solvents such as purified water).
(Item 73) The pharmaceutical composition of any one of items 1 to 60, 65 to 66 or 68, the granule for suspension of any one of items 61 to 64, the suspension of item 67, or the method of any one of claims 69 to 72, for treating or suppressing an oxidative stress disorder, modulating one or more energy biomarkers, normalizing one or more energy biomarkers, or enhancing one or more energy biomarkers.
(Item 74) The pharmaceutical composition of any one of items 1 to 60, 65, 66, or 68, the granule for suspension of any one of items 61 to 64, the suspension of item 67, or the method of any one of claims 69 to 72, for treating or suppressing an oxidative stress disorder selected from the group consisting of: a mitochondrial disorder; an inherited mitochondrial

disease; Alpers Disease; Barth syndrome; a Beta-oxidation Defect; Carnitine-Acyl-Carnitine Deficiency; Carnitine Deficiency; a Creatine Deficiency Syndrome; Co-Enzyme Q10 Deficiency; Complex I Deficiency; Complex II Deficiency; Complex III Deficiency; Complex IV Deficiency; Complex V Deficiency; COX Deficiency; chronic progressive external ophthalmoplegia (CPEO); CPT I Deficiency; CPT II Deficiency; Friedreich's Ataxia (FA); Glutaric Aciduria Type II; Kearns-Sayre Syndrome (KSS); Lactic Acidosis; Long-Chain Acyl-CoA Dehydrogenase Deficiency (LCAD); LCHAD; Leigh Syndrome; Leigh-like Syndrome; Leber's Hereditary Optic Neuropathy (LHON); Lethal Infantile Cardiomyopathy (LIC); Luft Disease; Multiple Acyl-CoA Dehydrogenase Deficiency (MAD); Medium-Chain Acyl-CoA Dehydrogenase Deficiency (MCAD); Mitochondrial Myopathy, Encephalopathy, Lactacidosis, Stroke (MELAS); Myoclonic Epilepsy with Ragged Red Fibers (MERRF); Mitochondrial Recessive Ataxia Syndrome (MIRAS); Mitochondrial Cytopathy, Mitochondrial DNA Depletion; Mitochondrial Encephalopathy; Mitochondrial Myopathy; Myoneurogastrointestina Disorder and Encephalopathy (MNGIE); Neuropathy, Ataxia, and Retinitis Pigmentosa (NARP); Pearson Syndrome; Pyruvate Carboxylase Deficiency; Pyruvate Dehydrogenase Deficiency; a POLG Mutation; a Respiratory Chain Disorder; Short-Chain Acyl-CoA Dehydrogenase Deficiency (SCAD); SCHAD; Very Long- Chain Acyl-CoA Dehydrogenase Deficiency (VLCAD); a myopathy; cardiomyopathy; encephalomyopathy; a neurodegenerative disease; Parkinson's disease; Alzheimer's disease; amyotrophic lateral sclerosis (ALS); a motor neuron disease; a neurological disease; epilepsy; an age-associated disease; macular degeneration; diabetes; metabolic syndrome; cancer; brain cancer; a genetic disease; Huntington's Disease; a mood disorder; schizophrenia; bipolar disorder; a pervasive developmental disorder; autistic disorder; Asperger's syndrome; childhood disintegrative disorder (CDD); Rett's disorder; PDD-not otherwise specified (PDD-NOS); a cerebrovascular accident; stroke; a vision impairment; optic neuropathy; dominant inherited juvenile optic atrophy; optic neuropathy caused by a toxic agent; glaucoma; Stargardt's macular dystrophy; diabetic retinopathy; diabetic maculopathy; retinopathy of prematurity; ischemic reperfusion-related retinal injury; oxygen poisoning; a haemoglobinopathy; thalassemia; sickle cell anemia; seizures; ischemia; renal tubular acidosis; attention deficit/hyperactivity disorder (ADHD); a neurodegenerative disorder resulting in hearing or balance impairment; Dominant Optic Atrophy (DOA); Maternally inherited diabetes and deafness (MIDD); chronic fatigue; contrast-induced kidney damage; contrast-induced retinopathy damage; Abetalipoproteinemia; retinitis pigmentosum; Wolfram's disease; Tourette syndrome; cobalamin c defect; methylmalonic aciduria; glioblastoma; Down's syndrome; acute tubular necrosis; a muscular dystrophy; a leukodystrophy; Progressive Supranuclear Palsy; spinal muscular atrophy; hearing loss; noise induced hearing loss; traumatic brain injury; Juvenile Huntington's Disease; Multiple Sclerosis; NGLY1; Multiple System Atrophy; Adrenoleukodystrophy; and Adrenomyeloneuropathy.

(Item 75) The pharmaceutical composition of any one of items 1 to 60, 65 to 66, or 68, the granule for suspension of any one of items 61-64, the suspension of item 67, or the method of any one of claims 69-72, for treating or suppressing amyotrophic lateral sclerosis (ALS).

Effects of the Invention

The present disclosure provides a granular agent for suspension with excellent properties of dispersibility and suppression of hydrate generation and crystal growth.

BRIEF DESCRIPTION OF DRAWINGS

[Figure 1] Figure **1** shows (top) 100x and (bottom) 700× microscope images of a crystal of an anhydrate of Example 12 stored for 3 days at 5°C. Since the size changes depending on the magnification of display of images or printing, reference can be taken from the dimensions in each image. The crystal of an anhydrate is a very thin rod-like crystal, with a degree of yellowness that is not high (light yellow color).

[Figure 2] Figure **2** shows (top) 100× and (bottom) 700× microscope images of a crystal of a hydrate of Example 16 stored for 14 days at 5°C. The crystal of a hydrate is a tabular or block-like crystal, with a degree of yellowness that is high. Some are found as a crystal formed from a combination of two pairs.

[Figure 3] Figure **3** shows pictures of each of, from the left, Example 14, Example 4, Example 20, Example 15, and Example 16 stored for 14 days at 5°C. Under visual inspection, a crystal of an hydrate is light yellow, and a crystal of a hydrate is yellow to orange. If a crystal that is yellow to orange is found from visual observation, the crystal is evaluated as "having a hydrate (⊙)".

DETAILED DESCRIPTION OF THE PREFERRED EMBODIMENTS

The present disclosure is described hereinafter in more detail. Throughout the entire specification, a singular

expression should be understood as encompassing the concept thereof in the plural form, unless specifically noted otherwise. Thus, singular articles (e.g., "a", "an", "the", etc. in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present invention pertains. In case of a contradiction, the present specification (including the definitions) takes precedence.

The present disclosure is further described in detail hereinafter.

The abbreviations used herein have the conventional meaning within the scope of the art unless specifically noted otherwise.

The term "about" for a value or parameter herein includes variations about the value or parameter itself. Unless specifically noted otherwise, "about X" for example includes "X" itself as well as values with an acceptable error of ± 10% therefrom.

As used herein, "Trolox" indicates 6-hydroxy-2,5,7,8-tetramethylchroman-2-carboxylic acid. R form is referred to as R Trolox, and S form is referred to as S Trolox. Trolox can be prepared by a synthesis method that is well known to those skilled in the art, such as the methods described in US Patent No. 3,947,473, US Patent No. 4,003,919, and US Patent No. 4,026,907.

As used herein, "binding agent" refers to an additive used for imparting a binding force to a powder mixture including an active pharmaceutical ingredient and additives for molding, which is an additive that uniforms the content by fixing the ratio of active pharmaceutical ingredient to additive in a formulation, or improves flowability by increasing the particle size of a formulation in a solid state. Examples thereof include cellulose binding agents, povidone binding agents, vinyl alcohol binding agents, thickening polysaccharide binding agents, etc.

Examples of cellulose binding agents include hydroxypropyl cellulose, hypromellose, methyl cellulose, hypromellose phthalate, hypromellose acetate succinate, carmellose, hydroxyethyl cellulose, alginic acid, etc.

Examples of povidone binding agents include povidone, copovidone, etc. Povidone is a linear polymer of 1-vinyl-2-pyrrolidone. Copovidone is a copolymer of 1-vinyl-2-pyrrolidone and vinyl acetate. Each of povidone and copovidone can be distinguished by the viscosity characteristic (K value). K values can be found by the method of measuring K values specified in the sections of "Povidone" and "Copovidone" in the Official Monograph of "Japanese Pharmacopoeia 18th Edition". Specifically for "povidone", weigh accurately an amount of Povidone, calculated on the anhydrous basis, specified in the following table, dissolve in water to make exactly 100 mL, allow to stand for 60 minutes, and use this solution as the sample solution. Perform the test with the sample solution and with water at 25°C as directed in Method 1 under the Viscosity Determination, specified in the Japanese Pharmacopoeia, at 25°C, and calculate the K value by the following formula.

[Formula 1]

$$K=\frac{1.5\log \nu_{rel.}-1}{0.15+0.003c}+\frac{\sqrt{300c\log \nu_{rel.}+(c+1.5c\log \nu_{rel.})^2}}{0.15c+0.003c^2}$$

$c$ : **mass (g) calculated on anhydrous basis in 100 mL of solution**

$\nu_{rel.}$ **: ratio of kinematic viscosity of sample solution to kinematic viscosity of water**

| Displayed K value | Amount (g) calculated on anhydrous basis |
|---|---|
| 18 or greater | 5.00 |
| Greater than 18 and equalto or less than 95 | 1.00 |
| Greater than 95 | 0.10 |

Specifically for "copovidone", weigh exactly an amount of Copovidone, equivalent to 1.00 g, calculated on the dried basis, and dissolve in water to make exactly 100 mL, allow to stand for 60 minutes, and use this solution as the sample solution. Perform the test with the sample solution and with water at 25°C as directed Method 1 under the Viscosity Determination, specified in the Japanese Pharmacopoeia, at 25°C and calculate the K value by the following formula.

[Formula 2]

$$K = \frac{1.5 \log \nu_{rel.} - 1}{0.15 + 0.003c} + \frac{\sqrt{300c \log \nu_{rel.} + (c + 1.5c \log \nu_{rel.})^2}}{0.15c + 0.003c^2}$$

**$c$ : mass (g) calculated on dried basis in 100 mL of solution**

**$\nu_{rel.}$ : ratio of kinematic viscosity of sample solution to kinematic viscosity of water**

"Nominal K value" is described on the present product. The nominal K value specified on the products of the raw materials that are used are described herein. Povidone and copovidone that can be used herein are not limited by such numerical values. "Nominal K value" is a representative value. In the case of Povidone, when actually measured using the present measurement method, the K value of Povidone having a nominal K value of 15 or less is in the range of not less than 85.0% and not more than 115.0% of the nominal K value, and the K value of Povidone having a nominal K value exceeding 15 is in the range of not less than 90.0% and not more 108.0% of the nominal K value. In the case of Copovidone, when actually measured using the present measurement method, the K value of Copovidone is in the range of not less than 90.0% and not more than 110.0% of the nominal K value. For example, if nominal K value of a raw material of Povidone is described as "17", the K value found by the present measurement method allows for 90.0% to 108.0% of the value thereof, i.e., "15.3 to 18.4".

The K value for povidone is, for example, 10 to 120, preferably 10 to 100, more preferably 10 to 80, still more preferably 15 to 35, and most preferably 25 to 35. Examples of povidone include Kollidon 17 PF (BASF) (nominal K value: 17), Plasdone K29/32 (Ashland) (nominal K value: 29 to 32), etc.

The K value for copovidone is, for example, 10 to 120, preferably 10 to 80, and more preferably 15 to 35. Examples of copovidone include Kollidon VA 64 (BASF) (nominal K value: 28), Plasdone S630 (Ashland) (nominal K value: 28), etc.

Examples of vinyl alcohol binding agents include polyvinyl alcohol, polyvinyl alcohol-polyethylene glycol graft copolymer, polyvinyl alcohol/acrylic acid/methyl methacrylate copolymer, etc.

Examples of thickening polysaccharide binding agents include xanthan gum, gellan gum, guar gum, carrageenan, gum arabic, pectin, pullulan, etc.

The content of "binding agent" is not particularly limited herein, but is, for example, 1 mg to 1000 mg, preferably 2 mg to 500 mg, more preferably 5 mg to 300 mg, and still more preferably 20 mg to 200 mg with respect to 1000 mg of a compound represented by formula 1 or a pharmaceutically acceptable salt or solvate thereof.

As used herein, "dispersant" refers to a substance that improves flowability or dispersion of particles in a solid state, prevents aggregation of particles with one another to a certain extent when a formulation contacts water, improves dispersion of formulation in water upon contact with water, or prevents firm re-aggregation of particles of a formulation in water with one another. Examples thereof include inorganic salt dispersants, ether dispersants, starch dispersants, cellulose dispersants, fatty acid ester salt dispersants, etc.

Examples of inorganic salt dispersants include light anhydrous silicic acid, magnesium aluminometasilicate, etc.

Examples of ether dispersants include polyethylene glycol (polyethylene glycol 600, polyethylene glycol 4000, polyethylene glycol 6000, etc.), propylene glycol, etc.

Examples of starch dispersants include corn starch, rice starch, sodium starch glycolate, partially pregelatinized starch, etc.

Examples of cellulose dispersants include crystalline cellulose/carmellose sodium, carmellose, carmellose calcium, etc.

Examples of fatty acid ester salt dispersants include sucrose fatty acid ester, sodium stearyl fumarate, etc.

The content of "dispersant" in the present disclosure is not particularly limited, but is, for example, 0.01 to 10% by weight, preferably 0.1 to 8% by weight, more preferably 0.5 to 5% by weight, still more preferably 1 to 5% by weight, and particularly preferably 2 to 5% by weight with respect to the weight of the entire composition.

As used herein, "suspending agent" refers to a substance that is added in order to maintain dispersion of suspended particles, improve ease of dosing, etc. when dispersing/suspending granules in water as a suspension. A substance with a property of increasing viscosity or a substance with viscosity can be used as a "suspending agent".

Examples of suspending agents include: thickening polysaccharides such as xanthan gum, guar gum, locust bean gum, tragacanth gum, sodium alginate, and carrageenan; acrylic acid copolymers such as carboxyvinyl polymer;

cellulose derivatives such as carboxymethyl cellulose, hypromellose, hydroxypropyl cellulose, and methyl cellulose; polyvinyl alcohol, povidone (polyvinyl pyrrolidone), combinations thereof, etc.

The content of "suspending agent" is not particularly limited herein, but is, for example, 0.1 to 600 mg, preferably 1.25 to 300 mg, more preferably 2.5 to 300 mg, and still more preferably 6.25 to 300 mg with respect to 1000 mg of a compound represented by formula 1 or a pharmaceutically acceptable salt or solvate thereof.

As used herein, "sweetener" is a corrigent for improving the ease of dosing of a formulation or masking an unpleasant taste of a component contained in a formulation by adding sweetness to the formulation.

Examples of sweeteners include aspartame, neotame, advantame, sucralose, sodium saccharin, dipotassium glycyrrhizate, acesulfame K, maltitol, erythritol, xylitol, trehalose, thaumatin, stevia, etc.

The content of "sweetener" is not particularly limited herein, and is desirably added at an amount sufficient to impart a suitable corrigent effect upon preparation of a suspension, but is, for example, 0.125 to 900 mg, preferably 1.25 to 600 mg, and more preferably 6.25 to 300 mg with respect to 1000 mg of a compound represented by formula 1 or a pharmaceutically acceptable salt or solvate thereof.

The pharmaceutical composition of the present disclosure may include an additive that can be used in a common formulation, as long as the effect of the invention is not obstructed. Examples of such additives include, but are not limited to, thickeners, preservatives, stabilizers, buffer, colorants, fragrances, etc.

The present disclosure encompasses all forms of (R)-2-hydroxy-2-methyl-4-(2,4,5-trimethyl-3,6-dioxocyclohexa-1,4-dienyl)butanamide (compound of formula 1) (e.g., tautomer, amorphous, crystalline, various crystalline polymorphisms, etc.) The present disclosure also encompasses enantiomers and mixtures thereof. The ratio of R forms for such mixtures is not particularly limited, but is preferably 60% by weight or greater, more preferably 80% by weight or greater, still more preferably 90% by weight or greater, particularly preferably 95% by weight or greater, and most preferably 99% by weight or greater.

Since a compound of formula 1 and pharmaceutically acceptable salt thereof may be present in a form of a hydrate and/or solvate, such hydrates and solvates such as ethanol solvates are also encompassed by the present disclosure.

Examples of "pharmaceutically acceptable salt" include, but are not limited to, alkali metal salts such as sodium salt and potassium salt; alkaline earth metal salts such as calcium salt and magnesium salt; inorganic metal salts such as zinc salt; organic basic salts such as triethylamine, triethanolamine, trihydroxymethylaminomethane, and amino acid; inorganic acid salts such as hydrochloride, hydrobromate, sulfate, phosphate, and nitrate; organic acid salts such as acetate, propionate, succinate, lactate, malate, tartrate, citrate, maleate, fumarate, methanesulfonate, p-toluenesulfonate, benzenesulfonate, and ascorbate, etc.

A compound of formula 1 used in the present disclosure may be crushed as needed.

In the present disclosure, "medical setting" refers to any site where the pharmaceutical compositions of this disclosure are used, including hospitals, clinics, nursing facilities, and homes (e.g., home healthcare).

"Modulation" of, or to "modulate," an energy biomarker means to change the level of the energy biomarker towards a desired value, or to change the level of the energy biomarker in a desired direction (e.g., increase or decrease). International Publication No. WO 2016/100579 is incorporated by reference. Modulation can include, but is not limited to, normalization and enhancement as defined below.

"Normalization" of, or to "normalize," an energy biomarker is defined as changing the level of the energy biomarker from a pathological value towards a normal value, where the normal value of the energy biomarker can be 1) the level of the energy biomarker in a healthy person or subject, or 2) a level of the energy biomarker that alleviates one or more undesirable symptoms in the person or subject. That is, to normalize an energy biomarker which is depressed in a disease state means to increase the level of the energy biomarker towards the normal (healthy) value or towards a value which alleviates an undesirable symptom; to normalize an energy biomarker which is elevated in a disease state means to decrease the level of the energy biomarker towards the normal (healthy) value or towards a value which alleviates an undesirable symptom.

"Enhancement" of, or to "enhance," energy biomarkers means to intentionally change the level of one or more energy biomarkers away from either the normal value, or the value before enhancement, in order to achieve a beneficial or desired effect. For example, in a situation where significant energy demands are placed on a subject, it may be desirable to increase the level of ATP in that subject to a level above the normal level of ATP in that subject. Enhancement can also be of beneficial effect in a subject suffering from a disease or pathology such as a mitochondrial disorder, in that normalizing an energy biomarker may not achieve the optimum outcome for the subject; in such cases, enhancement of one or more energy biomarkers can be beneficial, for example, higher-than-normal levels of ATP, or lower-than-normal levels of lactic acid (lactate) can be beneficial to such a subject.

By modulating, normalizing, or enhancing the energy biomarker Coenzyme Q means modulating, normalizing, or enhancing the variant or variants of Coenzyme Q which is predominant in the species of interest. For example, the variant of Coenzyme Q which predominates in humans is Coenzyme Q10. If a species or subject has more than one variants of Coenzyme Q present in significant amounts (i.e., present in amounts which, when modulated, normalized, or enhanced,

can have a beneficial effect on the species or subject), modulating, normalizing, or enhancing Coenzyme Q can refer to modulating, normalizing or enhancing any or all variants of Coenzyme Q present in the species or subject.

By the term "respiratory chain disorder", it means a disorder which results in the decreased utilization of oxygen by a mitochondrion, cell, tissue, or individual, due to a defect or disorder in a protein or other component contained in the mitochondrial respiratory chain. By "protein or other component contained in the mitochondrial respiratory chain", it means the components (including, but not limited to, proteins, tetrapyrroles, and cytochromes) comprising mitochondrial complex I, II, III, IV, and/or V. "Respiratory chain protein" refers to the protein components of those complexes, and "respiratory chain protein disorder" means a disorder which results in the decreased utilization of oxygen by a mitochondrion, cell, tissue, or individual, due to a defect or disorder in a protein contained in the mitochondrial respiratory chain.

The term "Parkinson's", (also called "Parkinsonism" and "Parkinsonian syndrome") ("PD") is intended to include not only Parkinson's disease but also drug-induced Parkinsonism and post-encephalitic Parkinsonism. Parkinson's disease is also known as paralysis agitans or shaking palsy. It is characterized by tremor, muscular rigidity and loss of postural reflexes. The disease usually progresses slowly with intervals of 10 to 20 years elapsing before the symptoms cause incapacity. Due to their mimicry of effects of Parkinson's disease, treatment of animals with methamphetamine or MPTP has been used to generate models for Parkinson's disease. These animal models have been used to evaluate the efficacy of various therapies for Parkinson's disease.

The term "Friedreich's ataxia" is intended to embrace other related ataxias, and is also sometimes referred to as hereditary ataxia, familial ataxia, or Friedreich's tabes.

The term "ataxia" is an aspecific clinical manifestation implying dysfunction of parts of the nervous system that coordinate movement, such as the cerebellum. People with ataxia have problems with coordination because parts of the nervous system that control movement and balance are affected. Ataxia may affect the fingers, hands, arms, legs, body, speech, and eye movements. The word ataxia is often used to describe a symptom of incoordination which can be associated with infections, injuries, other diseases, or degenerative changes in the central nervous system. Ataxia is also used to denote a group of specific degenerative diseases of the nervous system called the hereditary and sporadic ataxias. Ataxias are also often associated with hearing impairments.

There are three types of ataxia, cerebellar ataxia, including vestibulo-cerebellar dysfunction, spino-cerebellar dysfunction, and cerebro-cerebellar dysfunction; sensory ataxia; and vestibular ataxia. Examples of the diseases which are classifiable into spinocerebellar ataxia or multiple system atrophy are hereditary olivopontocerebellar atrophy, hereditary cerebellar cortical atrophy, Friedreich's ataxia, Machado-Joseph diseases, Ramsay Hunt syndrome, hereditary dentatorubral-pallidoluysian atrophy, hereditary spastic paraplegia, Shy-Drager syndrome, cortical cerebellar atrophy, striato-nigral degeneration, Marinesco-Sjogren syndrome, alcoholic cortical cerebellar atrophy, paraneoplastic cerebellar atrophy associated with malignant tumor, toxic cerebellar atrophy caused by toxic substances, Vitamin E deficiency due to mutation of a Tocopherol transfer protein (aTTP) or lipid absorption disorder such as Abetalipoproteinemia, cerebellar atrophy associated with endocrine disturbance and the like.

Examples of ataxia symptoms are motor ataxia, trunk ataxia, limb ataxia and the like, autonomic disturbance such as orthostatic hypotension, dysuria, hypohidrosis, sleep apnea, orthostatic syncope and the like, stiffness of lower extremity, ocular nystagmus, oculomotor nerve disorder, pyramidal tract dysfunction, extrapyramidal symptoms (postural adjustment dysfunction, muscular rigidity, akinesia, tremors), dysphagia, lingual atrophy, posterior funiculus symptom, muscle atrophy, muscle weakness, deep hyperreflexia, sensory disturbance, scoliosis, kyphoscoliosis, foot deformities, anarthria, dementia, manic state, decreased motivation for rehabilitation and the like.

(Preferred Embodiments)

One embodiment of the present disclosure provides a pharmaceutical composition including:

a compound represented by formula 1:

[Chemical Formula 3]

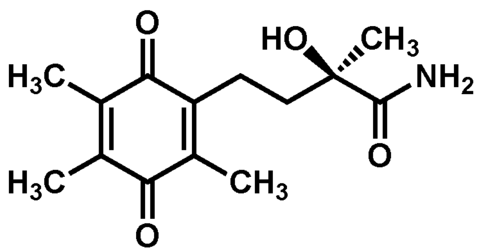

(compound 1) or a pharmaceutically acceptable salt or solvate thereof;
a binding agent; and
a dispersant.

In the present disclosure, the binding agent can include a binding agent selected from the group consisting of a cellulose binding agent, a povidone binding agent, a vinyl alcohol binding agent, and a thickening polysaccharide binding agent.

In the present disclosure, the cellulose binding agent can include a binding agent selected from the group consisting of hydroxypropyl cellulose, hypromellose, and methyl cellulose.

In the present disclosure, the povidone binding agent can include a binding agent selected from the group consisting of povidone and copovidone.

In the present disclosure, the vinyl alcohol binding agent can include a binding agent selected from the group consisting of polyvinyl alcohol and a polyvinyl alcohol-polyethylene glycol graft copolymer.

In the present disclosure, the thickening polysaccharide binding agent can include xanthan gum.

In the present disclosure, the dispersant can be selected from the group consisting of an inorganic salt dispersant, an ether dispersant, a starch dispersant, a cellulose dispersant, and a fatty acid ester salt dispersant.

In the present disclosure, the inorganic salt dispersant can include a dispersant selected from the group consisting of light anhydrous silicic acid and magnesium aluminometasilicate.

In the present disclosure, the ether dispersant can include polyethylene glycol 6000.

In the present disclosure, the starch dispersant can include corn starch.

In the present disclosure, the cellulose dispersant can include a dispersant selected from the group consisting of crystalline cellulose/carmellose sodium, carmellose, and carmellose calcium.

In the present disclosure, the fatty acid ester salt dispersant can include sodium stearyl fumarate.

In the present disclosure, the binding agent can include a cellulose binding agent, and
the dispersant can include a dispersant selected from the group consisting of an inorganic salt dispersant, an ether dispersant, a starch dispersant, a cellulose dispersant, and a fatty acid ester salt dispersant.

In the present disclosure, the binding agent can include a cellulose binding agent, and
the dispersant can include a dispersant selected from the group consisting of an inorganic salt dispersant, an ether dispersant, a starch dispersant, and a cellulose dispersant.

In the present disclosure, the binding agent can include a binding agent selected from the group consisting of hydroxypropyl cellulose, hypromellose, and methyl cellulose, and
the dispersant can include a dispersant selected from the group consisting of light anhydrous silicic acid, polyethylene glycol 6000, corn starch, carmellose, crystalline cellulose/carmellose sodium, magnesium aluminometasilicate, sodium stearyl fumarate, and carmellose calcium.

In the present disclosure, the binding agent can include a binding agent selected from the group consisting of hydroxypropyl cellulose, hypromellose, and methyl cellulose, and
the dispersant can include a dispersant selected from the group consisting of light anhydrous silicic acid, polyethylene glycol 6000, corn starch, and carmellose.

In the present disclosure, the binding agent can include hydroxypropyl cellulose, and
the dispersant can include a dispersant selected from the group consisting of light anhydrous silicic acid, corn starch, carmellose, polyethylene glycol 6000, crystalline cellulose/carmellose sodium, magnesium aluminometasilicate, sodium stearyl fumarate, and carmellose calcium.

In the present disclosure, the binding agent can include hydroxypropyl cellulose, and
the dispersant can include a dispersant selected from the group consisting of light anhydrous silicic acid, corn starch, and carmellose.

In the present disclosure, the binding agent can include hydroxypropyl cellulose, and
the dispersant can include light anhydrous silicic acid.

In the present disclosure, the binding agent can include hypromellose, and
the dispersant can include a dispersant selected from the group consisting of a light anhydrous silicic acid dispersant and polyethylene glycol 6000.

In the present disclosure, the binding agent can include hypromellose, and
the dispersant can include light anhydrous silicic acid.

In the present disclosure, the binding agent can include methyl cellulose, and
the dispersant can include light anhydrous silicic acid.

In the present disclosure, the binding agent can include a povidone binding agent, and
the dispersant can include a dispersant selected from the group consisting of an inorganic salt dispersant, an ether dispersant, a starch dispersant, a cellulose dispersant, and a fatty acid ester salt dispersant.

In the present disclosure, the binding agent can include a povidone binding agent, and

the dispersant can include a dispersant selected from the group consisting of an inorganic salt dispersant and a cellulose dispersant.

In the present disclosure, the binding agent can include copovidone, and
the dispersant can include a dispersant selected from the group consisting of light anhydrous silicic acid and crystalline cellulose/carmellose sodium.

In the present disclosure, the binding agent can include a cellulose binding agent, and
the dispersant can include an inorganic salt dispersant.

In the present disclosure, the binding agent can include a binding agent selected from the group consisting of hydroxypropyl cellulose, hypromellose, and methyl cellulose, and
the dispersant can include a dispersant selected from the group consisting of light anhydrous silicic acid and magnesium aluminometasilicate.

In the present disclosure, the binding agent can include a binding agent selected from the group consisting of hydroxypropyl cellulose, hypromellose, and methyl cellulose, and
the dispersant can include light anhydrous silicic acid.

In the present disclosure, the binding agent can include a binding agent selected from the group consisting of hydroxypropyl cellulose and hypromellose, and
the dispersant can include light anhydrous silicic acid.

In the present disclosure, the binding agent can include a cellulose binding agent, and
the dispersant can include an ether dispersant.

In the present disclosure, the binding agent can include a binding agent selected from the group consisting of hydroxypropyl cellulose and hypromellose, and
the dispersant can include polyethylene glycol 6000.

In the present disclosure, the binding agent can include a hypromellose, and
the dispersant can include polyethylene glycol 6000.

In the present disclosure, the binding agent can include a cellulose binding agent, and the dispersant can include a starch dispersant.

In the present disclosure, the binding agent can include hydroxypropyl cellulose, and
the dispersant can include corn starch.

In the present disclosure, the binding agent can include a cellulose binding agent, and
the dispersant can include a cellulose dispersant.

In the present disclosure, the binding agent can include hydroxypropyl cellulose, and
the dispersant can include a dispersant selected from carmellose, crystalline cellulose/carmellose sodium, and carmellose calcium.

In the present disclosure, the binding agent can include hydroxypropyl cellulose, and
the dispersant can include a dispersant selected from crystalline cellulose/carmellose sodium and carmellose calcium.

In the present disclosure, the binding agent can include hydroxypropyl cellulose, and
the dispersant can include carmellose.

In the present disclosure, the binding agent can include a cellulose binding agent, and
the dispersant can include a fatty acid ester salt dispersant.

In the present disclosure, the binding agent can include hydroxypropyl cellulose, and
the dispersant can include sodium stearyl fumarate.

In the present disclosure, the binding agent can include a povidone binding agent, and
the dispersant can include a cellulose dispersant.

In the present disclosure, the binding agent can include copovidone, and
the dispersant can include light anhydrous silicic acid.

In the present disclosure, the binding agent can include a povidone binding agent, and
the dispersant can include an inorganic salt dispersant.

In the present disclosure, the binding agent can include a binding agent selected from the group consisting of copovidone, povidone (nominal K value: 29 to 32), and povidone (nominal K value: 17), and
the dispersant can include light anhydrous silicic acid.

In the present disclosure, the binding agent can include a binding agent selected from the group consisting of copovidone and povidone (nominal K value: 29 to 32), and
the dispersant can include light anhydrous silicic acid.

In the present disclosure, the binding agent can include a vinyl alcohol binding agent, and
the dispersant can include an inorganic salt dispersant.

In the present disclosure, the binding agent can include a binding agent selected from the group consisting of polyvinyl alcohol and a polyvinyl alcohol/polyethylene glycol/graft copolymer, and
the dispersant can include light anhydrous silicic acid.

In the present disclosure, the binding agent can include polyvinyl alcohol/polyethylene glycol/graft copolymer, and

the dispersant can include light anhydrous silicic acid.

In the present disclosure, the binding agent can include a vinyl alcohol binding agent, and

the dispersant can include a cellulose dispersant.

In the present disclosure, the binding agent can include a binding agent selected from the group consisting of polyvinyl alcohol and a polyvinyl alcohol/polyethylene glycol/graft copolymer, and

the dispersant can include crystalline cellulose/carmellose sodium.

In the present disclosure, the binding agent can include a thickening polysaccharide binding agent, and

the dispersant can include an inorganic salt dispersant.

In the present disclosure, the binding agent can include xanthan gum, and

the dispersant can include light anhydrous silicic acid.

In the present disclosure, the pharmaceutical composition can include a suspending agent.

In the present disclosure, the suspending agent can include a carboxyvinyl polymer.

In the present disclosure, the pharmaceutical composition can include a sweetener.

In the present disclosure, the sweetener can include sucralose.

In the present disclosure, the pharmaceutical composition can include 1 mg to 1000 mg of binding agent with respect to 1000 mg of a compound represented by formula 1 or a pharmaceutically acceptable salt or solvate thereof.

In the present disclosure, the pharmaceutical composition can include a dispersant at 0.01 to 10% by weight with respect to the weight of the entire composition.

In the present disclosure, the pharmaceutical composition can include a 1 mg to 1000 mg of suspending agent with respect to 1000 mg of a compound represented by formula 1 or a pharmaceutically acceptable salt or solvate thereof.

In the present disclosure, the pharmaceutical composition can include 1 mg to 1000 mg of sweetener with respect to 1000 mg of a compound represented by formula 1 or a pharmaceutically acceptable salt or solvate thereof.

One embodiment of the present disclosure provides a granule for suspension for the manufacture of a pharmaceutical composition, including the pharmaceutical composition of any of the preceding embodiments.

In the present disclosure, the granule for suspension can be a granule for suspension for the preparation of a suspension.

One embodiment of the present disclosure provides a granule for suspension for the manufacture of a pharmaceutical composition, wherein the granule for suspension is the pharmaceutical composition of any one of the preceding embodiments.

In the present disclosure, the granule for suspension can be a granule for suspension for the preparation of a suspension.

One embodiment of the present disclosure provides the pharmaceutical composition of any one of the preceding embodiments, which is a granule for suspension for the manufacture of a pharmaceutical composition.

In the present disclosure, the pharmaceutical composition can be a granule for suspension for the preparation of a suspension.

One embodiment of the present disclosure provides a suspension including the pharmaceutical composition of any one of the preceding embodiments, which is a granule for suspension for the manufacture of a pharmaceutical composition.

In the present disclosure, the pharmaceutical composition can be in a suspended state.

One embodiment of the present disclosure provides a method of treating or preventing a disease in a subject in need thereof, comprising administering to the subject in need thereof an effective amount of a pharmaceutical composition of any one of preceding embodiments or a suspension of preceding embodiments.

One embodiment of the present disclosure provides a method for producing a pharmaceutical composition of any one of preceding embodiments or a suspension of preceding embodiments, comprising mixing said compound or a pharmaceutically acceptable salt or solvate thereof, said binding agent and a dispersant.

One embodiment of the present disclosure provides a method for producing a pharmaceutical composition, comprising mixing the granules for suspension of any one of preceding embodiments with necessary additives and/or suspension solvents (e.g., including water solvents such as purified water).

One embodiment of the present disclosure provides a method of preparing a pharmaceutical composition for administration to a subject in a medical setting, comprising mixing the granules for suspension of any one of preceding embodiments with necessary additives and/or suspension solvents (e.g., including water solvents such as purified water).

One embodiment of the present disclosure provides the pharmaceutical composition of any one of preceding embodiments, the granule for suspension of any one of preceding embodiments, the suspension of any one of preceding embodiments, or the method of any one of preceding embodiments, for treating or suppressing an oxidative stress disorder, modulating one or more energy biomarkers, normalizing one or more energy biomarkers, or enhancing one or more energy biomarkers.

One embodiment of the present disclosure provides the pharmaceutical composition of any one of preceding embodiments, the granule for suspension of any one of preceding embodiments, or the method of any one of preceding embodiments, for treating or suppressing an oxidative stress disorder selected from the group consisting of: a mitochondrial disorder; an inherited mitochondrial disease; Alpers Disease; Barth syndrome; a Beta-oxidation Defect; Carnitine-Acyl-Carnitine Deficiency; Carnitine Deficiency; a Creatine Deficiency Syndrome; Co-Enzyme Q10 Deficiency; Complex I Deficiency; Complex II Deficiency; Complex III Deficiency; Complex IV Deficiency; Complex V Deficiency; COX Deficiency; chronic progressive external ophthalmoplegia (CPEO); CPT I Deficiency; CPT II Deficiency; Friedreich's Ataxia (FA); Glutaric Aciduria Type II; Kearns-Sayre Syndrome (KSS); Lactic Acidosis; Long-Chain Acyl-CoA Dehydrogenase Deficiency (LCAD); LCHAD; Leigh Syndrome; Leigh-like Syndrome; Leber's Hereditary Optic Neuropathy (LHON); Lethal Infantile Cardiomyopathy (LIC); Luft Disease; Multiple Acyl-CoA Dehydrogenase Deficiency (MAD); Medium-Chain Acyl-CoA Dehydrogenase Deficiency (MCAD); Mitochondrial Myopathy, Encephalopathy, Lactacidosis, Stroke (MELAS); Myoclonic Epilepsy with Ragged Red Fibers (MERRF); Mitochondrial Recessive Ataxia Syndrome (MIRAS); Mitochondrial Cytopathy, Mitochondrial DNA Depletion; Mitochondrial Encephalopathy; Mitochondrial Myopathy; Myoneurogastrointestina Disorder and Encephalopathy (MNGIE); Neuropathy, Ataxia, and Retinitis Pigmentosa (NARP); Pearson Syndrome; Pyruvate Carboxylase Deficiency; Pyruvate Dehydrogenase Deficiency; a POLG Mutation; a Respiratory Chain Disorder; Short-Chain Acyl-CoA Dehydrogenase Deficiency (SCAD); SCHAD; Very Long- Chain Acyl-CoA Dehydrogenase Deficiency (VLCAD); a myopathy; cardiomyopathy; encephalomyopathy; a neurodegenerative disease; Parkinson's disease; Alzheimer's disease; amyotrophic lateral sclerosis (ALS); a motor neuron disease; a neurological disease; epilepsy; an age-associated disease; macular degeneration; diabetes; metabolic syndrome; cancer; brain cancer; a genetic disease; Huntington's Disease; a mood disorder; schizophrenia; bipolar disorder; a pervasive developmental disorder; autistic disorder; Asperger's syndrome; childhood disintegrative disorder (CDD); Rett's disorder; PDD-not otherwise specified (PDD-NOS); a cerebrovascular accident; stroke; a vision impairment; optic neuropathy; dominant inherited juvenile optic atrophy; optic neuropathy caused by a toxic agent; glaucoma; Stargardt's macular dystrophy; diabetic retinopathy; diabetic maculopathy; retinopathy of prematurity; ischemic reperfusion-related retinal injury; oxygen poisoning; a haemoglobinopathy; thalassemia; sickle cell anemia; seizures; ischemia; renal tubular acidosis; attention deficit/hyperactivity disorder (ADHD); a neurodegenerative disorder resulting in hearing or balance impairment; Dominant Optic Atrophy (DOA); Maternally inherited diabetes and deafness (MIDD); chronic fatigue; contrast-induced kidney damage; contrast-induced retinopathy damage; Abetalipoproteinemia; retinitis pigmentosum; Wolfram's disease; Tourette syndrome; cobalamin c defect; methylmalonic aciduria; glioblastoma; Down's syndrome; acute tubular necrosis; a muscular dystrophy; a leukodystrophy; Progressive Supranuclear Palsy; spinal muscular atrophy; hearing loss; noise induced hearing loss; traumatic brain injury; Juvenile Huntington's Disease; Multiple Sclerosis; NGLY1; Multiple System Atrophy; Adrenoleukodystrophy; and Adrenomyeloneuropathy.

One embodiment of the present disclosure provides the pharmaceutical composition of any one of preceding embodiments, the suspension agent of any one of preceding embodiments, the granule for suspension of any one of preceding embodiments, or the method of any one of preceding embodiments, for treating or suppressing amyotrophic lateral sclerosis (ALS).

Diseases amenable to treatment or suppression with compositions and methods of the present disclosure

A variety of disorders/diseases are believed to be caused or aggravated by oxidative stress affecting normal electron flow in the cells, such as mitochondrial disorders, impaired energy processing disorders, neurodegenerative diseases and diseases of aging, and can be treated or suppressed using the composition of the present disclosure.

Non-limiting examples of oxidative stress disorders include, for example, mitochondrial disorders (including inherited mitochondrial diseases) such as Alpers Disease, Barth syndrome, Beta-oxidation Defects, Carnitine-Acyl-Carnitine Deficiency, Carnitine Deficiency, Creatine Deficiency Syndromes, Co-Enzyme Q10 Deficiency, Complex I Deficiency, Complex II Deficiency, Complex III Deficiency, Complex IV Deficiency, Complex V Deficiency, COX Deficiency, chronic progressive external ophthalmoplegia (CPEO), CPT I Deficiency, CPT II Deficiency, Friedreich's Ataxia (FA), Glutaric Aciduria Type II, Kearns-Sayre Syndrome (KSS), Lactic Acidosis, Long-Chain Acyl-CoA Dehydrogenase Deficiency (LCAD), LCHAD, Leigh Disease or Syndrome, Leigh-like Syndrome, Leber's Hereditary Optic Neuropathy (LHON, also referred to as Leber's Disease, Leber's Optic Atrophy (LOA), or Leber's Optic Neuropathy (LON)), Lethal Infantile

Cardiomyopathy (LIC), Luft Disease, Multiple Acyl-CoA Dehydrogenase Deficiency (MAD), Medium-Chain Acyl-CoA Dehydrogenase Deficiency (MCAD), Mitochondrial Myopathy, Encephalopathy, Lactacidosis, Stroke (MELAS), Myoclonic Epilepsy with Ragged Red Fibers (MERRF), Mitochondrial Recessive Ataxia Syndrome (MIRAS), Mitochondrial Cytopathy, Mitochondrial DNA Depletion, Mitochondrial Encephalopathy, Mitochondrial Myopathy, Myoneurogastrointestinal Disorder and Encephalopathy (MNGIE), Neuropathy, Ataxia, and Retinitis Pigmentosa (NARP), Pearson Syndrome, Pyruvate Carboxylase Deficiency, Pyruvate Dehydrogenase Deficiency, POLG Mutations, Respiratory Chain Disorder, Short-Chain Acyl-CoA Dehydrogenase Deficiency (SCAD), SCHAD, Very Long-Chain Acyl-CoA Dehydrogenase Deficiency (VLCAD); myopathies such as cardiomyopathy and encephalomyopathy; neurodegenerative diseases such as Parkinson's disease, Alzheimer's disease, and amyotrophic lateral sclerosis (ALS, also known as Lou Gehrig's disease); motor neuron diseases; neurological diseases such as epilepsy; age- associated diseases, particularly diseases for which CoQIO has been proposed for treatment, such as macular degeneration, diabetes (e.g. Type 2 diabetes mellitus), metabolic syndrome, and cancer (e.g. brain cancer); genetic diseases such as Huntington's Disease (which is also a neurological disease); mood disorders such as schizophrenia and bipolar disorder; pervasive developmental disorders such as autistic disorder, Asperger's syndrome, childhood disintegrative disorder (CDD), Rett's disorder, and PDD-not otherwise specified (PDD-NOS); cerebrovascular accidents such as stroke; vision impairments such as those caused by neurodegenerative diseases of the eye such as optic neuropathy, Leber's hereditary optic neuropathy, dominant inherited juvenile optic atrophy, optic neuropathy caused by toxic agents, glaucoma, age-related macular degeneration (both "dry" or non-exudative macular degeneration and "wet" or exudative macular degeneration), Stargardt's macular dystrophy, diabetic retinopathy, diabetic maculopathy, retinopathy of prematurity, or ischemic reperfusion-related retinal injury; disorders caused by energy impairment include diseases due to deprivation, poisoning or toxicity of oxygen, and qualitative or quantitative disruption in the transport of oxygen such as haemoglobionopathies, for example thalassemia or sickle cell anemia; other diseases in which mitochondrial dysfunction is implicated such as excitoxic, neuronal injury, such as that associated with seizures, stroke and ischemia; and other disorders including renal tubular acidosis; attention deficit/hyperactivity disorder (ADHD); neurodegenerative disorders resulting in hearing or balance impairment; Dominant Optic Atrophy (DOA); Maternally inherited diabetes and deafness (MIDD); chronic fatigue; contrast-induced kidney damage; contrast-induced retinopathy damage; Abetalipoproteinemia; retinitis pigmentosum; Wolfram's disease; Tourette syndrome; cobalamin c defect; methylmalonic aciduria; glioblastoma; Down's syndrome; acute tubular necrosis; muscular dystrophies; leukodystrophies; Progressive Supranuclear Palsy; spinal muscular atrophy; hearing loss (e.g. noise induced hearing loss); traumatic brain injury; Juvenile Huntington's Disease; Multiple Sclerosis; NGLY1; Multiple System Atrophy; Adrenoleukodystrophy; and Adrenomyeloneuropathy. It is to be understood that certain specific diseases or disorders may fall within more than one category; for example, Huntington's Disease is a genetic disease as well as a neurological disease. Furthermore, certain oxidative stress diseases and disorders may also be considered mitochondrial disorders. International Publication No. WO 2016/100579 is incorporated by reference.

A compound represented by formula 1 (compound 1) has an unpleasant taste, which is masked with a sweetener in the present formulation. The particles are bound with a binding agent and prepared as a granule (large particle) for manufacturability and handling during distribution, and flowability is imparted with a dispersant. Dispersibility is imparted with a dispersant for suspension in water. When a formulation with such features was designed, growth in size of suspended particles/hydration of a drug were observed over time after preparing a suspension.

Such a change impairs the property of a formulation. Growth in size of suspended particles reduces the dispersion stability and drug uniformity in a suspension associated therewith, and hydration of a drug results in discoloration and a change in the outer appearance/growth in size of particles through the growth of a hydrate. An increase in size of a particle or drug has an adverse effect on ease of oral administration (foreign body sensation, etc.) and causes clogging of a feeding tube in administration therethrough in a patient being treated via gastrostomy tube.

The pharmaceutical composition of the present disclosure has excellent dispersibility that is beneficial upon preparation of a suspension, and suppressed hydrate generation and crystal growth, which is beneficial over time after preparation of a suspension.

(Formulation method)

A course crystal of compound 1 is crushed as needed to obtain compound 1 for the preparation of a formulation. While the active pharmaceutical ingredient/raw material is also generally crushed as needed in order to design a homogenous and uniform formulation, the active pharmaceutical ingredient/raw material may be used directly when there is no issue in terms of the design of the formulation. Compound 1 can be obtained by, for example, the method described in WO 2021/167095.

Specific examples of the preparation method of the formulation are described below; however, the preparation method of the formulation is not limited thereto. Compound 1, a binding agent, a suspending agent, and a sweetener are mixed as the raw materials of a formulation. The mixture is loaded into a preparation mixer. Purified water is gradually added as water for granulation, and the mixture was mixed/agitated, while repeating mixing/agitation of the mixture and addition of purified water, to obtain granules. The granules are subjected to wet sieving through a sieve and dried to obtain dried granules. The dried granules are subjected to dry sieving through a sieve to obtain granules.

Granules and a dispersant are mixed, and the entire amount is sifted with a sieve. The sifted product is mixed to obtain a formulation.

In addition to the preparation method described above, the formulation can also be prepared by, for example, the following method. Compound 1, a suspending agent, a sweetener, and, if necessary, a binding agent are mixed as formulation raw materials. This mixture is charged into a stirring granulator, purified water or an aqueous binding agent

solution is added, and the mixture is stirred and granulated to obtain granules. The granules are subjected to wet sieving, if necessary, and dried with a fluidized bed granulator or the like to obtain dried granules. The dried granules are subjected to dry sieving as necessary to obtain granules. A formulation can be obtained by mixing the granules and a dispersant.

As the granulation method, a wet granulation method is preferable, and examples thereof include stirring granulation method, fluidized bed granulation method, extrusion granulation method, and kneading granulation method. Examples of a method for adding a binding agent include adding same in the form of a powder together with formulation raw materials, and adding a binding agent by dissolving same in a solvent as a granulation liquid when granulating other formulation raw materials.

[Examples]

The present invention is described hereinafter in more detail with the Examples, Reference Examples, Test Examples, etc., but the present invention is not limited thereto.

The following Examples, Reference Examples, etc. used the following carboxyvinyl polymer, sucralose, hydroxypropyl cellulose, hypromellose, methyl cellulose, povidone, copovidone, polyvinyl alcohol, polyvinyl alcohol/polyethylene glycol/graft copolymer, xanthan gum, light anhydrous silicic acid, magnesium aluminometasilicate, polyethylene glycol 6000, corn starch, crystalline cellulose/carmellose sodium, carmellose, carmellose calcium, and sodium stearyl fumarate, but the present invention is not limited thereto.

*Carboxyvinyl polymer: Carbopol 971P (Lubrizol)
*Sucralose: Sucralose (Merck)
*Hydroxypropyl cellulose: HPC-L fine (Nippon Soda Co., Ltd.)
*Hypromellose: TC-5R (Shin-Etsu Chemical Co., Ltd.)
*Methyl cellulose: SM-4 (Shin-Etsu Chemical Co., Ltd.)
*Povidone (nominal K value: 17): Kollidon 17 PF (BASF)
*Povidone (nominal K value: 29 to 32): Plasdone K29/32 (Ashland)
*Copovidone: Kollidon VA 64 (BASF)
*Polyvinyl alcohol: GOHSENOL EG-05PW (Mitsubishi Chemical Corporation)
*Polyvinyl alcohol/polyethylene glycol/graft copolymer: Kollicoat IR (BASF)
*Xanthan gum: Keltrol T (Sumitomo Pharma Food & Chemical Co., Ltd.)
*Light anhydrous silicic acid: AEROSIL 200 (EVONIK)
*Magnesium aluminometasilicate: Neusilin FL2 (Fuji Chemical Industries Co., Ltd.)
*Polyethylene glycol 6000: Macrogol 6000 (SP) (Sanyo Chemical Industries, Ltd.)
*Corn starch: Nihon Shokuhin Pharmacopoeia Corn Starch XX16 (Nihon Shokuhin Kako Co., Ltd.)
*Crystalline cellulose/carmellose sodium: Ceolus RC-A591NF (Asahi Kasei Corporation)
*Carmellose: NS-300 (Gotoku Chemical Company Ltd.)
*Carmellose calcium: E.C.G-505 (Gotoku Chemical Company Ltd.)
*Sodium stearyl fumarate: PRUV (Ashland)

The purified water used in the manufacture of a formulation was "Japanese Pharmacopoeia purified water" (Takasugi Pharmaceutical Co., Ltd.).

Examples 1 to 21 and Reference Example 1

The formulations of Examples 1 to 21 and Reference Example 1 were prepared by the following method by using compound 1 and additives at the amounts specified in the following table.

(1) Preparation of compound 1

Uncrushed compound 1 was crushed using a pulverizer QUADROCOMIL (Powrex Corp) in order to adjust crude crystals of compound 1 to a suitable particle size to obtain compound 1 for the preparation of a formulation.

(2) Preparation of granule A

Compound 1 (60g), hydroxypropyl cellulose (9.9 g), carboxyvinyl polymer (0.6 g), and sucralose (1.5 g) were placed in a plastic bag as the raw materials of a formulation and mixed therein. The mixture was loaded into a preparation mixer YM-Q-7 (DALTON Corporation). Purified water (20.5 g) was gradually added as water for granulation while repeating mixing/agitation of the mixture and addition of purified water. The mixture was then agitated and mixed for 3

minutes with the preparation mixture to obtain granules. The granules were subjected to wet sieving through a metal sieve with an aperture size of 710 μm, expanded on a paper plate, and dried for 16 hours or more at 40°C with a heat dryer (laboratory glassware dryer DRU600TB/Advantec) to obtain dried granules. The dried granules were subjected to dry sieving through a metal sieve with an aperture size of 850 μm to obtain granule A.

(3) Preparation of granule B

Granules were prepared by the same preparation method as granule A, other than using compound 1 (18 g), hypromellose (2.97 g), carboxyvinyl polymer (0.18 g), and sucralose (0.45 g) as the raw materials of a formulation and purified water (3.6 g) as water for granulation, to obtain granule B.

(4) Preparation of granule C

Granules were prepared by the same preparation method as granule A, other than using compound 1 (18 g), methyl cellulose (2.97 g), carboxyvinyl polymer (0.18 g), and sucralose (0.45 g) as the raw materials of a formulation and purified water (2.8 g) as water for granulation, to obtain granule C.

(5) Preparation of granule D

Granules were prepared by the same preparation method as granule A, other than using compound 1 (18 g), povidone (nominal K value: 29 to 32) (2.97 g), carboxyvinyl polymer (0.18 g), and sucralose (0.45 g) as the raw materials of a formulation and purified water (2.3 g) as water for granulation, to obtain granule D.

(6) Preparation of granule E

Granules were prepared by the same preparation method as granule A, other than using compound 1 (18 g), carboxyvinyl polymer (0.18 g), and sucralose (0.45 g) as the raw materials of a formulation and purified water (2.6 g) as water for granulation, to obtain granule E.

(7) Preparation of granule F

Granules were prepared by the same preparation method as granule A, other than using compound 1 (18 g), polyvinyl alcohol (2.97 g), carboxyvinyl polymer (0.18 g), and sucralose (0.45 g) as the raw materials of a formulation and purified water (4.1 g) as water for granulation, to obtain granule F.

(8) Preparation of granule G

Granules were prepared by the same preparation method as granule A, other than using compound 1 (18 g), polyvinyl alcohol/polyethylene glycol/graft copolymer (2.97 g), carboxyvinyl polymer (0.18 g), and sucralose (0.45 g) as the raw materials of a formulation and purified water (2.7 g) as water for granulation, to obtain granule G.

(9) Preparation of granule H

Granules were prepared by the same preparation method as granule A, other than using compound 1 (18 g), copovidone (2.97 g), carboxyvinyl polymer (0.18 g), and sucralose (0.45 g) as the raw materials of a formulation and purified water (2.5 g) as water for granulation, to obtain granule H.

(10) Preparation of granule I

Granules were prepared by the same preparation method as granule A, other than using compound 1 (18 g), povidone (nominal K value: 17) (2.97 g), carboxyvinyl polymer (0.18 g), and sucralose (0.45 g) as the raw materials of a formulation and purified water (2.5 g) as water for granulation, to obtain granule I.

(11) Preparation of granule J

Granules were prepared by the same preparation method as granule A, other than using compound 1 (18 g), xanthan gum (2.97 g), carboxyvinyl polymer (0.18 g), and sucralose (0.45 g) as the raw materials of a formulation and purified water (2.5 g) as water for granulation, to obtain granule J.

(12) Preparation of granule K

Granules were prepared by the same preparation method as granule A, other than using compound 1 (18 g) and hydroxypropyl cellulose (2.97 g) as the raw materials of a formulation and purified water (2.9 g) as water for granulation, to obtain granule K.

(13) Preparation of Example 1

Granule A (7.2 g) and light anhydrous silicic acid (0.3 g) were placed in a plastic bag and mixed therein. The entire amount was sifted through a metal sieve with an aperture size of 850 μm. The sifted product was retrieved in a plastic bag and mixed therein to obtain a formulation of Example 1.

(14) Preparation of Example 2

Granule B (7.2 g) and light anhydrous silicic acid (0.3 g) were placed in a plastic bag and mixed therein. The entire amount was sifted through a metal sieve with an aperture size of 850 μm. The sifted product was retrieved in a plastic bag and mixed therein to obtain a formulation of Example 2.

(15) Preparation of Example 3

Granule C (7.2 g) and light anhydrous silicic acid (0.3 g) were placed in a plastic bag and mixed therein. The entire amount was sifted through a metal sieve with an aperture size of 850 μm. The sifted product was retrieved in a plastic bag and mixed therein to obtain a formulation of Example 3.

(16) Preparation of Example 4

Granule H (7.2 g) and light anhydrous silicic acid (0.3 g) were placed in a plastic bag and mixed therein. The entire amount was sifted through a metal sieve with an aperture size of 850 μm. The sifted product was retrieved in a plastic bag and mixed therein to obtain a formulation of Example 4.

(17) Preparation of Example 5

Granule B (7.2 g) and polyethylene glycol 6000 (0.3 g) were placed in a plastic bag and mixed therein. The entire amount was sifted through a metal sieve with an aperture size of 850 μm. The sifted product was retrieved in a plastic bag and mixed therein to obtain a formulation of Example 5.

(18) Preparation of Example 6

Granule A (7.2 g) and corn starch (0.3 g) were placed in a plastic bag and mixed therein. The entire amount was sifted through a metal sieve with an aperture size of 850 μm. The sifted product was retrieved in a plastic bag and mixed therein to obtain a formulation of Example 6.

(19) Preparation of Example 7

Granule A (7.2 g) and carmellose (0.3 g) were placed in a plastic bag and mixed therein. The entire amount was sifted through a metal sieve with an aperture size of 850 μm. The sifted product was retrieved in a plastic bag and mixed therein to obtain a formulation of Example 7.

(20) Preparation of Example 8

Granule H (7.2 g) and crystalline cellulose/carmellose sodium (0.3 g) were placed in a plastic bag and mixed therein. The entire amount was sifted through a metal sieve with an aperture size of 850 μm. The sifted product was retrieved in a plastic bag and mixed therein to obtain a formulation of Example 8.

(21) Preparation of Example 9

Granule A (7.2 g) and polyethylene glycol 6000 (0.3 g) were placed in a plastic bag and mixed therein. The entire amount was sifted through a metal sieve with an aperture size of 850 μm. The sifted product was retrieved in a plastic bag

and mixed therein to obtain a formulation of Example 9.

(22) Preparation of Example 10

Granule A (7.2 g) and crystalline cellulose/carmellose sodium (0.3 g) were placed in a plastic bag and mixed therein. The entire amount was sifted through a metal sieve with an aperture size of 850 μm. The sifted product was retrieved in a plastic bag and mixed therein to obtain a formulation of Example 10.

(23) Preparation of Example 11

Granule A (7.2 g) and sodium stearyl fumarate (0.3 g) were placed in a plastic bag and mixed therein. The entire amount was sifted through a metal sieve with an aperture size of 850 μm. The sifted product was retrieved in a plastic bag and mixed therein to obtain a formulation of Example 11.

(24) Preparation of Example 12

Granule A (7.2 g) and carmellose calcium (0.3 g) were placed in a plastic bag and mixed therein. The entire amount was sifted through a metal sieve with an aperture size of 850 μm. The sifted product was retrieved in a plastic bag and mixed therein to obtain a formulation of Example 12.

(25) Preparation of Example 13

Granule F (7.2 g) and light anhydrous silicic acid (0.3 g) were placed in a plastic bag and mixed therein. The entire amount was sifted through a metal sieve with an aperture size of 850 μm. The sifted product was retrieved in a plastic bag and mixed therein to obtain a formulation of Example 13.

(26) Preparation of Example 14

Granule G (7.2 g) and light anhydrous silicic acid (0.3 g) were placed in a plastic bag and mixed therein. The entire amount was sifted through a metal sieve with an aperture size of 850 μm. The sifted product was retrieved in a plastic bag and mixed therein to obtain a formulation of Example 14.

(27) Preparation of Example 15

Granule J (7.2 g) and light anhydrous silicic acid (0.3 g) were placed in a plastic bag and mixed therein. The entire amount was sifted through a metal sieve with an aperture size of 850 μm. The sifted product was retrieved in a plastic bag and mixed therein to obtain a formulation of Example 15.

(28) Preparation of Example 16

Granule F (7.2 g) and crystalline cellulose/carmellose sodium (0.3 g) were placed in a plastic bag and mixed therein. The entire amount was sifted through a metal sieve with an aperture size of 850 μm. The sifted product was retrieved in a plastic bag and mixed therein to obtain a formulation of Example 16.

(29) Preparation of Example 17

Granule G (7.2 g) and crystalline cellulose/carmellose sodium (0.3 g) were placed in a plastic bag and mixed therein. The entire amount was sifted through a metal sieve with an aperture size of 850 μm. The sifted product was retrieved in a plastic bag and mixed therein to obtain a formulation of Example 17.

(30) Preparation of Example 18

Granule A (7.2 g) and magnesium aluminometasilicate (0.3 g) were placed in a plastic bag and mixed therein. The entire amount was sifted through a metal sieve with an aperture size of 850 μm. The sifted product was retrieved in a plastic bag and mixed therein to obtain a formulation of Example 18.

(31) Preparation of Example 19

Granule D (7.2 g) and light anhydrous silicic acid (0.3 g) were placed in a plastic bag and mixed therein. The entire amount was sifted through a metal sieve with an aperture size of 850 μm. The sifted product was retrieved in a plastic bag and mixed therein to obtain a formulation of Example 19.

(32) Preparation of Example 20

Granule I (7.2 g) and light anhydrous silicic acid (0.3 g) were placed in a plastic bag and mixed therein. The entire amount was sifted through a metal sieve with an aperture size of 850 μm. The sifted product was retrieved in a plastic bag and mixed therein to obtain a formulation of Example 20.

(33) Preparation of Example 21

Granule K (6.99 g) and light anhydrous silicic acid (0.3 g) were placed in a plastic bag and mixed therein. The entire amount was sifted through a metal sieve with an aperture size of 850 μm. The sifted product was retrieved in a plastic bag and mixed therein to obtain a formulation of Example 21.

(34) Preparation of Reference Example 1

Granule E (6.21 g) was placed in a plastic bag and mixed therein to obtain a formulation of Reference Example 1.

[Table 1]

| Amounts of components per 1000 mg of compound 1 | | | | | | | | | | | | |
|---|---|---|---|---|---|---|---|---|---|---|---|---|
| Component | Example 1 | Example 2 | Example 3 | Example 4 | Example 5 | Example 6 | Example 7 | Example 8 | Example 9 | Example 10 | Example 11 | Example 12 |
| Compound 1 | 1000 | 1000 | 1000 | 1000 | 1000 | 1000 | 1000 | 1000 | 1000 | 1000 | 1000 | 1000 |
| Carboxyvinyl polymer | 10 | 10 | 10 | 10 | 10 | 10 | 10 | 10 | 10 | 10 | 10 | 10 |
| Sucralose | 25 | 25 | 25 | 25 | 25 | 25 | 25 | 25 | 25 | 25 | 25 | 25 |
| Hydroxypropyl cellulose | 165 | | | | | 165 | 165 | | 165 | 165 | 165 | 165 |
| Hypromellose | | 165 | | | 165 | | | | | | | |
| Methyl cellulose | | | 165 | | | | | | | | | |
| Polyvidone (nominal K value: 29 to 32) | | | | | | | | | | | | |
| Copovidone | | | | 165 | | | | 165 | | | | |
| Polyvinyl alcohol | | | | | | | | | | | | |
| Polyvinyl alcohol/polyethylene glycol/-graft copolymer | | | | | | | | | | | | |
| Polyvidone (nominal K value: 29 to 32) | | | | | | | | | | | | |
| Xanthan gum | | | | | | | | | | | | |
| Light anhydrous silicic acid | 50 | 50 | 50 | 50 | | | | | | | | |
| Polyethylene glycol 6000 | | | | | 50 | | | | 50 | | | |
| Crystalline cellulose/carmellose sodium | | | | | | | | 50 | | 50 | | |
| Corn starch | | | | | | 50 | | | | | | |
| Magnesium aluminometasilicate | | | | | | | | | | | | |

(continued)

| Amounts of components per 1000 mg of compound 1 | | | | | | | | | | | | |
|---|---|---|---|---|---|---|---|---|---|---|---|---|
| Component | Example 1 | Example 2 | Example 3 | Example 4 | Example 5 | Example 6 | Example 7 | Example 8 | Example 9 | Example 10 | Example 11 | Example 12 |
| Sodium stearyl fumarate | | | | | | | | | | | 50 | |
| Carmellose | | | | | | | 50 | | | | | |
| Carmellose calcium | | | | | | | | | | | | 50 |

[Table 2]

| Amounts of components per 1000 mg of compound 1 | | | | | | | | | | |
|---|---|---|---|---|---|---|---|---|---|---|
| Component | Example 13 | Example 14 | Example 15 | Example 16 | Example 17 | Example 18 | Example 19 | Example 20 | Example 21 | Reference Example 1 |
| Compound 1 | 1000 | 1000 | 1000 | 1000 | 1000 | 1000 | 1000 | 1000 | 1000 | 1000 |
| Carboxyvinyl polymer | 10 | 10 | 10 | 10 | 10 | 10 | 10 | 10 | | 10 |
| Sucralose | 25 | 25 | 25 | 25 | 25 | 25 | 25 | 25 | | 25 |
| Hydroxypropyl cellulose | | | | | | 165 | | | 165 | |
| Hypromellose | | | | | | | | | | |
| Methyl cellulose | | | | | | | | | | |
| Polyvidone (nominal K value: 29 to 32) | | | | | | | 165 | | | |
| Copovidone | | | | | | | | | | |
| Polyvinyl alcohol | 165 | | | 165 | | | | | | |
| Polyvinyl alcohol/polyethylene glycol/graft copolymer | | 165 | | | 165 | | | | | |
| Polyvidone (nominal K value: 17) | | | | | | | | 165 | | |
| Xanthan gum | | | 165 | | | | | | | |
| Light anhydrous silicic acid | 50 | 50 | 50 | | | | 50 | 50 | 50 | |
| Polyethylene glycol 6000 | | | | | | | | | | |
| Crystalline cellulose/carmellose sodium | | | | 50 | 50 | | | | | |
| Corn starch | | | | | | | | | | |
| Magnesium aluminometasilicate | | | | | | 50 | | | | |
| Sodium stearyl fumarate | | | | | | | | | | |
| Carmellose | | | | | | | | | | |
| Carmellose calcium | | | | | | | | | | |

Test Example 1 (Water dispersion of prepared formulation)

Ease of suspension when the present agent is administered as a suspension was evaluated by using water dispersion of the formulation as an indicator. The specific evaluation method is described below.

Each formulation (Examples 1 to 21 and Reference Example 1) at an amount corresponding to 1000 mg of compound 1 was measured out in a 20 mL volume transparent glass bottle (screw cap bottle 20 ml **No.** 5/Maruemu Corporation). **8.8** mL of purified water was placed therein and a cap (screw cap **No.** 5/Maruemu Corporation) was placed thereon. Immediately thereafter, the mixture was agitated up and down with one hand so that the container would be inverted 60 times. The dispersion of the formulations was studied. The evaluation criteria for dispersion are specified below. The test was conducted for each formulation at n = 3 to evaluate the mean score thereof.

<Evaluation criteria>

Scores: Evaluation
5: Homogenously dispersed
4: Homogenously dispersed, without large and crude particles that can pose a problem in oral administration or adhesion to the inner walls of the bottle
3: Dispersed, without large and crude particles that can pose a problem in oral administration or adhesion to the inner walls of the bottle
2: Small amount of large and crude particles that can pose a problem in oral administration or adhesion to the inner walls of the bottle, requiring additional agitation
1: Many instances of large and crude particles that can pose a problem in oral administration or adhesion to the inner walls of the bottle, requiring additional agitation

Test Example 2 (crystal growth and hydrate generation after preparation of a suspension)

The present agent was evaluated with respect to storage stability upon preparation of the present agent as a suspension by using crystal growth and hydrate generation of compound 1 in a suspension formulation as indicators. The specific method is described below.

Each formulation (Examples 1 to 21 and Reference Example 1) at an amount corresponding to 1000 mg of compound 1 was measured out in a 20 mL volume transparent glass bottle (screw cap bottle 20 ml No. 5/Maruemu Corporation). 8.8 mL of purified water was placed therein and a cap (screw cap No. 5/Maruemu Corporation) was placed thereon. Immediately thereafter, the mixture was dispersed by agitating the container up and down with one hand so that the container would be inverted 60 times. Samples with insufficient dispersion at this time were further agitated to prepare a suspension.

The formulation prepared as a suspension was stored for 3 days, 7 days, 14 days, or 1 month at 5°C. The extent of crystal growth and hydrate generation during each storage period was evaluated by visual inspection of the outer appearance and microscopic observation. The evaluation criteria of crystal growth and hydrate generation are specified below.

<Evaluation criteria>

Symbol: Evaluation
×: crystal growth and hydrate generation were not found through visual inspection of the outer appearance or microscopic observation
O: crystal growth and hydrate generation were not found through visual inspection of the outer appearance, but found through microscopic observation
⊙: crystal growth and hydrate generation were found through visual inspection of the outer appearance and microscopic observation

Thus, evaluation of × is the best evaluation.

[Table 3-1]

| Results of each test for each formulation | | | | | |
|---|---|---|---|---|---|
| Formulation | Test Example 1 | Test Example 2 | | | |
| | Water dispersion | Crystal growth and hydrate generation after storage | | | |
| | | 3 days | 7 days | 14 days | 1 month |
| Example 1 | 5 | × | × | × | × |
| Example 2 | 5 | × | × | × | × |
| Example 3 | 4 | × | × | × | × |
| Example 4 | 5 | × | × | × | ○ |
| Example 5 | 5 | × | × | × | × |
| Example 6 | 5 | × | × | × | ○ |
| Example 7 | 4 | × | × | × | ○ |
| Example 8 | 5 | × | × | × | ○ |
| Example 9 | 5 | × | × | ○ | ○ |
| Example 10 | 5 | × | × | ○ | ○ |

[Table 3-2]

| Formulation | Test Example 1 | Test Example 2 | | | |
|---|---|---|---|---|---|
| | Water dispersion | Crystal growth and hydrate generation after storage | | | |
| | | 3 days | 7 days | 14 days | 1 month |
| Example 11 | 5 | × | × | ○ | ⊙ |
| Example 12 | 5 | × | × | ○ | ⊙ |
| Example 13 | 3 | × | ○ | ○ | ⊙ |
| Example 14 | 5 | × | ○ | ⊙ | ⊙ |
| Example 15 | 3 | × | ○ | ⊙ | ⊙ |
| Example 16 | 5 | × | ○ | ⊙ | ⊙ |
| Example 17 | 5 | × | ○ | ○ | ⊙ |
| Example 18 | 2 | × | × | ○ | ⊙ |
| Example 19 | 5 | × | × | × | ○ |
| Example 20 | 5 | × | ○ | ○ | ⊙ |
| Example 21 | 5 | × | × | ○ | ⊙ |

[Table 3-3]

| Formulation | Test Example 1 | Test Example 2 | | | |
|---|---|---|---|---|---|
| | Water dispersion | Crystal growth and hydrate generation after storage | | | |
| | | 3 days | 7 days | 14 days | 1 month |
| Reference Example 1 | 5 | × | × | × | × |

Formulations, which have a score in Test Example 1 of 2 or higher and hydrate generation in Test Example 2 of × even at 3 days, are used as the Examples.

INDUSTRIAL APPLICABILITY

The present disclosure provides a pharmaceutical composition.

This application is based on a patent application No. 2022-144675 filed in Japan (filing date: September 12, 2022), the contents of which are incorporated in full herein.

## Claims

1. A pharmaceutical composition comprising:

    a compound represented by formula 1:

    [Chemical Formula 1]

    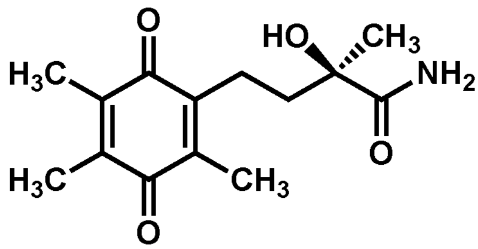

    or a pharmaceutically acceptable salt or solvate thereof;
    a binding agent; and
    a dispersant.

2. The pharmaceutical composition of claim **1,** wherein the binding agent comprises a binding agent selected from the group consisting of a cellulose binding agent, a povidone binding agent, a vinyl alcohol binding agent, and a thickening polysaccharide binding agent.

3. The pharmaceutical composition of claim 2, wherein the cellulose binding agent comprises a binding agent selected from the group consisting of hydroxypropyl cellulose, hypromellose, and methyl cellulose.

4. The pharmaceutical composition of claim 2, wherein the povidone binding agent comprises a binding agent selected from the group consisting of povidone and copovidone.

5. The pharmaceutical composition of claim 2, wherein the vinyl alcohol binding agent comprises a binding agent selected from the group consisting of polyvinyl alcohol and a polyvinyl alcohol-polyethylene glycol graft copolymer.

6. The pharmaceutical composition of claim 2, wherein the thickening polysaccharide binding agent comprises xanthan gum.

7. The pharmaceutical composition of any one of claims 1 to 6, wherein the dispersant is selected from the group consisting of an inorganic salt dispersant, an ether dispersant, a starch dispersant, a cellulose dispersant, and a fatty acid ester salt dispersant.

8. The pharmaceutical composition of claim 7, wherein the inorganic salt dispersant comprises a dispersant selected from the group consisting of light anhydrous silicic acid and magnesium aluminometasilicate.

9. The pharmaceutical composition of claim 7, wherein the ether dispersant comprises polyethylene glycol 6000.

10. The pharmaceutical composition of claim 7, wherein the starch dispersant comprises corn starch.

11. The pharmaceutical composition of claim 7, wherein the cellulose dispersant comprises a dispersant selected from the group consisting of crystalline cellulose/carmellose sodium, carmellose, and carmellose calcium.

**12.** The pharmaceutical composition of claim 7, wherein the fatty acid ester salt dispersant comprises sodium stearyl fumarate.

**13.** The pharmaceutical composition of claim 1, wherein

the binding agent comprises a cellulose binding agent, and
the dispersant comprises a dispersant selected from the group consisting of an inorganic salt dispersant, an ether dispersant, a starch dispersant, a cellulose dispersant, and a fatty acid ester salt dispersant.

**14.** The pharmaceutical composition of claim **1,** wherein

the binding agent comprises a cellulose binding agent, and
the dispersant comprises a dispersant selected from the group consisting of an inorganic salt dispersant, an ether dispersant, a starch dispersant, and a cellulose dispersant.

**15.** The pharmaceutical composition of claim **1,** wherein

the binding agent comprises a binding agent selected from the group consisting of hydroxypropyl cellulose, hypromellose, and methyl cellulose, and
the dispersant comprises a dispersant selected from the group consisting of light anhydrous silicic acid, polyethylene glycol 6000, corn starch, carmellose, crystalline cellulose/carmellose sodium, magnesium aluminometasilicate, sodium stearyl fumarate, and carmellose calcium.

**16.** The pharmaceutical composition of claim **1,** wherein

the binding agent comprises a binding agent selected from the group consisting of hydroxypropyl cellulose, hypromellose, and methyl cellulose, and
the dispersant comprises a dispersant selected from the group consisting of light anhydrous silicic acid, polyethylene glycol 6000, corn starch, and carmellose.

**17.** The pharmaceutical composition of claim **1,** wherein

the binding agent comprises hydroxypropyl cellulose, and
the dispersant comprises a dispersant selected from the group consisting of light anhydrous silicic acid, corn starch, carmellose, polyethylene glycol 6000, crystalline cellulose/carmellose sodium, magnesium aluminometasilicate, sodium stearyl fumarate, and carmellose calcium.

**18.** The pharmaceutical composition of claim **1,** wherein

the binding agent comprises hydroxypropyl cellulose, and
the dispersant comprises a dispersant selected from the group consisting of light anhydrous silicic acid, corn starch, and carmellose.

**19.** The pharmaceutical composition of claim **1,** wherein

the binding agent comprises hydroxypropyl cellulose, and
the dispersant comprises light anhydrous silicic acid.

**20.** The pharmaceutical composition of claim **1,** wherein

the binding agent comprises hypromellose, and
the dispersant comprises a dispersant selected from the group consisting of light anhydrous silicic acid and polyethylene glycol 6000.

**21.** The pharmaceutical composition of claim 1, wherein

the binding agent comprises hypromellose, and
the dispersant comprises light anhydrous silicic acid.

**22.** The pharmaceutical composition of claim 1, wherein

the binding agent comprises methyl cellulose, and
the dispersant comprises light anhydrous silicic acid.

**23.** The pharmaceutical composition of claim **1,** wherein

the binding agent comprises a povidone binding agent, and
the dispersant comprises a dispersant selected from the group consisting of an inorganic salt dispersant, an ether dispersant, a starch dispersant, a cellulose dispersant, and a fatty acid ester salt dispersant.

**24.** The pharmaceutical composition of claim 1, wherein

the binding agent comprises a povidone binding agent, and
the dispersant comprises a dispersant selected from the group consisting of an inorganic salt dispersant and a cellulose dispersant.

**25.** The pharmaceutical composition of claim 1, wherein

the binding agent comprises copovidone, and
the dispersant comprises a dispersant selected from the group consisting of light anhydrous silicic acid and crystalline cellulose/carmellose sodium.

**26.** The pharmaceutical composition of claim 1, wherein

the binding agent comprises a cellulose binding agent, and
the dispersant comprises an inorganic salt dispersant.

**27.** The pharmaceutical composition of claim 26, wherein

the binding agent comprises a binding agent selected from the group consisting of hydroxypropyl cellulose, hypromellose, and methyl cellulose, and
the dispersant comprises a dispersant selected from the group consisting of light anhydrous silicic acid and magnesium aluminometasilicate.

**28.** The pharmaceutical composition of claim 27, wherein

the binding agent comprises a binding agent selected from the group consisting of hydroxypropyl cellulose, hypromellose, and methyl cellulose, and
the dispersant comprises light anhydrous silicic acid.

**29.** The pharmaceutical composition of claim 28, wherein

the binding agent comprises a binding agent selected from the group consisting of hydroxypropyl cellulose and hypromellose, and
the dispersant comprises light anhydrous silicic acid.

**30.** The pharmaceutical composition of claim 1, wherein

the binding agent comprises a cellulose binding agent, and
the dispersant comprises an ether dispersant.

**31.** The pharmaceutical composition of claim 30, wherein

the binding agent comprises a binding agent selected from the group consisting of hydroxypropyl cellulose and hypromellose, and
the dispersant comprises polyethylene glycol 6000.

**32.** The pharmaceutical composition of claim 31, wherein

the binding agent comprises hypromellose, and
the dispersant comprises polyethylene glycol 6000.

**33.** The pharmaceutical composition of claim 1, wherein

the binding agent comprises a cellulose binding agent, and
the dispersant comprises a starch dispersant.

**34.** The pharmaceutical composition of claim 33, wherein

the binding agent comprises hydroxypropyl cellulose, and
the dispersant comprises corn starch.

**35.** The pharmaceutical composition of claim 1, wherein

the binding agent comprises a cellulose binding agent, and
the dispersant comprises a cellulose dispersant.

**36.** The pharmaceutical composition of claim 35, wherein

the binding agent comprises hydroxypropyl cellulose, and
the dispersant comprises a dispersant selected from carmellose, crystalline cellulose/carmellose sodium, and carmellose calcium.

**37.** The pharmaceutical composition of claim 36, wherein

the binding agent comprises hydroxypropyl cellulose, and
the dispersant comprises a dispersant selected from crystalline cellulose/carmellose sodium and carmellose calcium.

**38.** The pharmaceutical composition of claim 36, wherein

the binding agent comprises hydroxypropyl cellulose, and
the dispersant comprises carmellose.

**39.** The pharmaceutical composition of claim 1, wherein

the binding agent comprises a cellulose binding agent, and
the dispersant comprises a fatty acid ester salt dispersant.

**40.** The pharmaceutical composition of claim 39, wherein

the binding agent comprises hydroxypropyl cellulose, and
the dispersant comprises sodium stearyl fumarate.

**41.** The pharmaceutical composition of claim 1, wherein

the binding agent comprises a povidone binding agent, and
the dispersant comprises a cellulose dispersant.

**42.** The pharmaceutical composition of claim 41, wherein

the binding agent comprises copovidone, and
the dispersant comprises crystalline cellulose/carmellose sodium.

**43.** The pharmaceutical composition of claim 1, wherein

the binding agent comprises a povidone binding agent, and
the dispersant comprises an inorganic salt dispersant.

**44.** The pharmaceutical composition of claim 43, wherein

the binding agent comprises a binding agent selected from the group consisting of copovidone, povidone (nominal K value: 29 to 32), and povidone (nominal K value: 17), and
the dispersant comprises light anhydrous silicic acid.

**45.** The pharmaceutical composition of claim 44, wherein

the binding agent comprises a binding agent selected from the group consisting of copovidone and povidone (nominal K value: 29 to 32), and
the dispersant comprises light anhydrous silicic acid.

**46.** The pharmaceutical composition of claim 1, wherein

the binding agent comprises a vinyl alcohol binding agent, and
the dispersant comprises an inorganic salt dispersant.

**47.** The pharmaceutical composition of claim 46, wherein

the binding agent comprises a binding agent selected from the group consisting of polyvinyl alcohol and a polyvinyl alcohol/polyethylene glycol/graft copolymer, and
the dispersant comprises light anhydrous silicic acid.

**48.** The pharmaceutical composition of claim 47, wherein

the binding agent comprises a polyvinyl alcohol/polyethylene glycol/graft copolymer, and
the dispersant comprises light anhydrous silicic acid.

**49.** The pharmaceutical composition of claim 1, wherein

the binding agent comprises a vinyl alcohol binding agent, and
the dispersant comprises a cellulose dispersant.

**50.** The pharmaceutical composition of claim 49, wherein

the binding agent comprises a binding agent selected from the group consisting of polyvinyl alcohol and a polyvinyl alcohol/polyethylene glycol/graft copolymer, and
the dispersant comprises crystalline cellulose/carmellose sodium.

**51.** The pharmaceutical composition of claim 1, wherein

the binding agent comprises a thickening polysaccharide binding agent, and
the dispersant comprises an inorganic salt dispersant.

**52.** The pharmaceutical composition of claim 51, wherein

the binding agent comprises xanthan gum, and
the dispersant comprises light anhydrous silicic acid.

**53.** The pharmaceutical composition of any one of claims 1 to 52, wherein the pharmaceutical composition comprises a suspending agent.

**54.** The pharmaceutical composition of claim 53, wherein the suspending agent comprises a carboxyvinyl polymer.

**55.** The pharmaceutical composition of any one of claims 1 to 54, wherein the pharmaceutical composition comprises a

sweetener.

**56.** The pharmaceutical composition of claim 55, wherein the sweetener comprises sucralose.

**57.** The pharmaceutical composition of any one of claims 1 to 56, wherein the pharmaceutical composition comprises 1 mg to 1000 mg of binding agent with respect to 1000 mg of a compound represented by formula I or a pharmaceutically acceptable salt or solvate thereof.

**58.** The pharmaceutical composition of any one of claims 1 to 57, wherein the pharmaceutical composition comprises a dispersant at 0.01 to 10% by weight with respect to the weight of the entire composition.

**59.** The pharmaceutical composition of any one of claims 1 to 58, wherein the pharmaceutical composition comprises 1 mg to 1000 mg of suspending agent with respect to 1000 mg of a compound represented by formula I or a pharmaceutically acceptable salt or solvate thereof.

**60.** The pharmaceutical composition of any one of claims 1 to 59, wherein the pharmaceutical composition comprises 1 mg to 1000 mg of sweetener with respect to 1000 mg of a compound represented by formula I or a pharmaceutically acceptable salt or solvate thereof.

**61.** A granule for suspension for the manufacture of a pharmaceutical composition, comprising the pharmaceutical composition of any one of claims 1 to 60.

**62.** The granule for suspension of claim 61, which is a granule for suspension for the preparation of a suspension.

**63.** A granule for suspension for the manufacture of a pharmaceutical composition, wherein the granule for suspension is the pharmaceutical composition of any one of claims 1 to 60.

**64.** The granule for suspension of claim 63, which is a granule for suspension for the preparation of a suspension.

**65.** The pharmaceutical composition of any one of claims 1 to 60, which is a granule for suspension for the manufacture of a pharmaceutical composition.

**66.** The pharmaceutical composition of claim 65, which is a granule for suspension for the preparation of a suspension.

**67.** A suspension comprising the pharmaceutical composition of any one of claims 1 to 60, which is a granule for suspension for the manufacture of a pharmaceutical composition.

**68.** The pharmaceutical composition of any one of claims 1 to 60, which is in a suspended state.

FIG.1

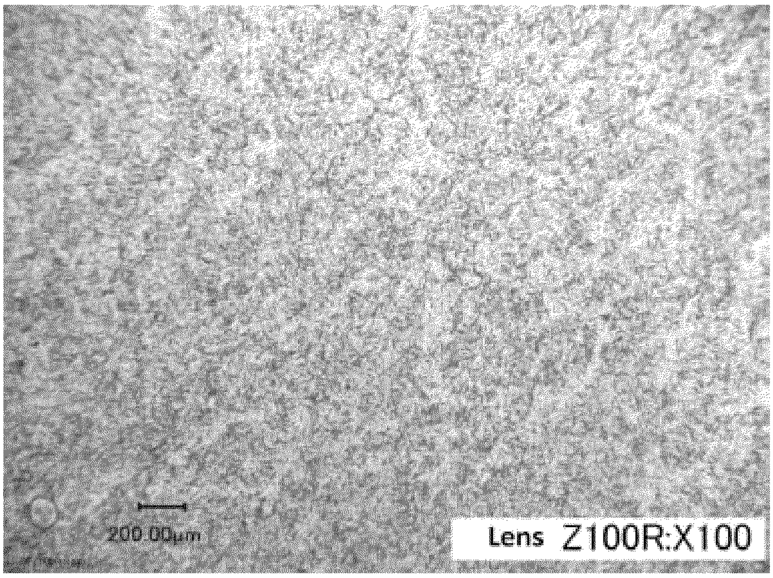


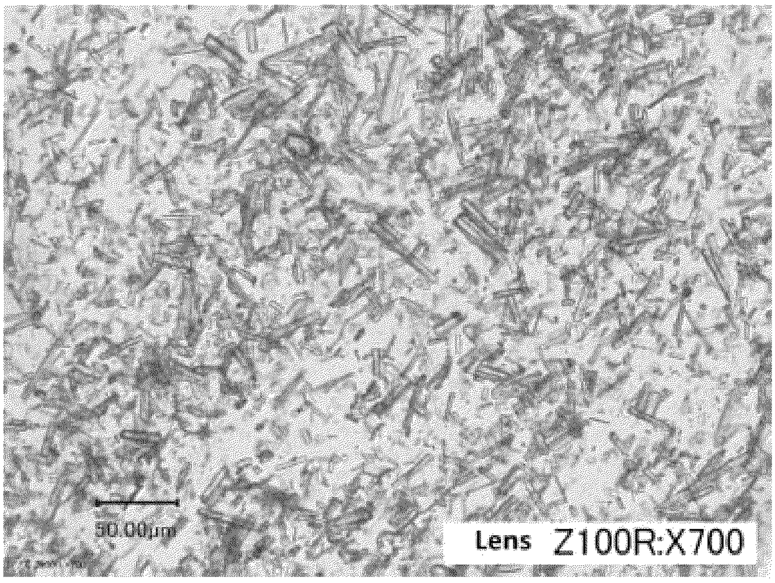

FIG.2

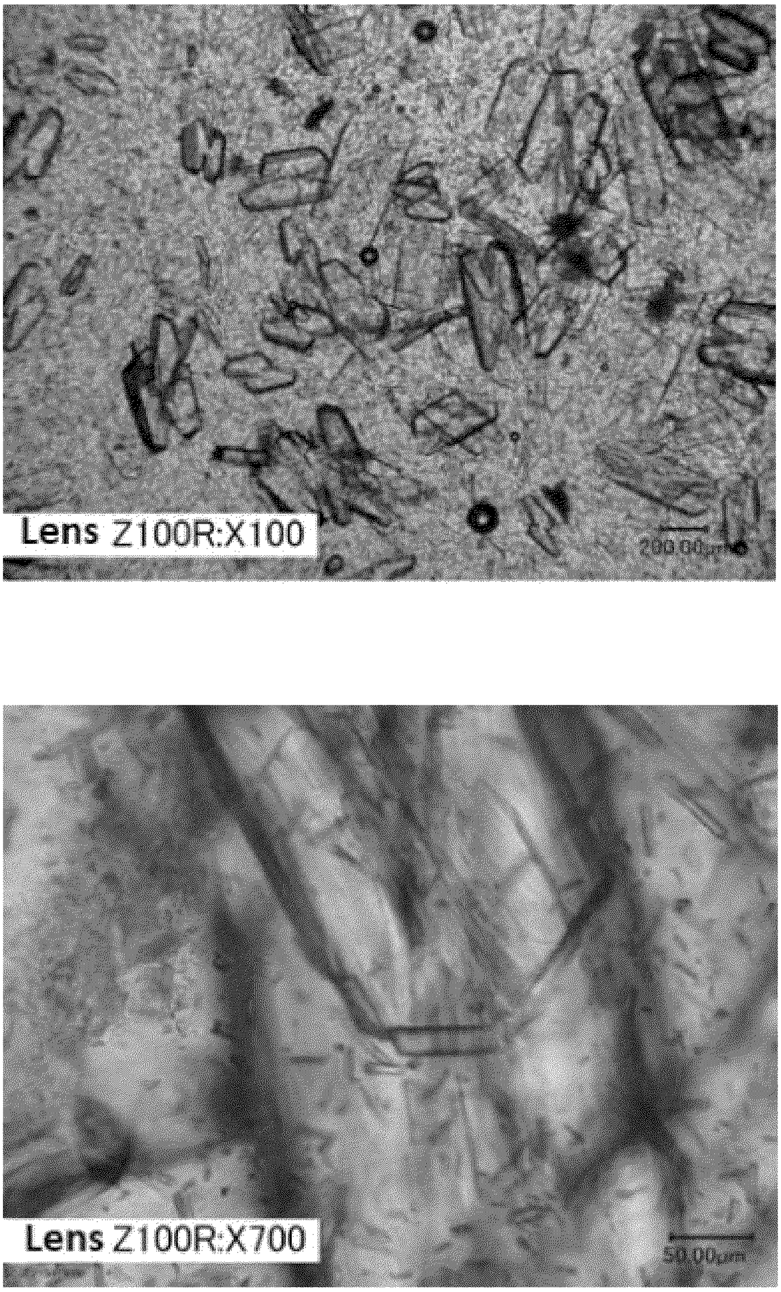

FIG.3

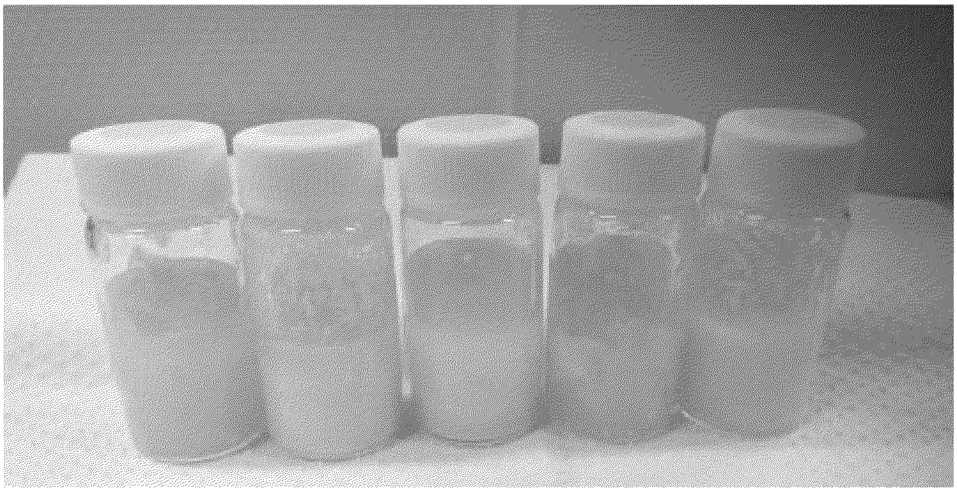

**INTERNATIONAL SEARCH REPORT**

International application No. **PCT/JP2023/032990**

**A. CLASSIFICATION OF SUBJECT MATTER**

***A61K 31/16***(2006.01)i; ***A61K 9/107***(2006.01)i; ***A61K 9/14***(2006.01)i; ***A61K 47/04***(2006.01)i; ***A61K 47/10***(2017.01)i; ***A61K 47/14***(2017.01)i; ***A61K 47/32***(2006.01)i; ***A61K 47/34***(2017.01)i; ***A61K 47/36***(2006.01)i; ***A61K 47/38***(2006.01)i
FI: A61K31/16; A61K9/107; A61K9/14; A61K47/04; A61K47/10; A61K47/14; A61K47/32; A61K47/34; A61K47/36; A61K47/38

According to International Patent Classification (IPC) or to both national classification and IPC

**B. FIELDS SEARCHED**

Minimum documentation searched (classification system followed by classification symbols)

A61K31/16; A61K9/107; A61K9/14; A61K47/04; A61K47/10; A61K47/14; A61K47/32; A61K47/34; A61K47/36; A61K47/38

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| Y | JP 2018-500326 A (BIOELECTRON TECHNOLOGY CORP.) 11 January 2018 (2018-01-11) claims, paragraphs [0172]-[0180] | 1-68 |
| Y | WO 2010/087447 A1 (MEIJI SEIKA KAISHA, LTD.) 05 August 2010 (2010-08-05) paragraphs [0049]-[0054] | 1-68 |
| Y | JP 2007-332097 A (TOWA YAKUHIN KK) 27 December 2007 (2007-12-27) paragraphs [0009]-[0012] | 1-68 |
| Y | JP 2006-306766 A (NIKKEN CHEM. CO., LTD.) 09 November 2006 (2006-11-09) paragraph [0017] | 1-68 |
| A | NODA, N. et al. Phase 1 Study to Investigate the Safety, Tolerability, and Pharmacokinetics of EPI-589 in Healthy Participants. Clinical Pharmacology in Drug Development. 21 August 2022, vol. 11, pp. 1136-1146 abstract | 1-68 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 November 2023** | **28 November 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2023/032990**

| Patent document cited in search report | Publication date (day/month/year) | Patent family member(s) | Publication date (day/month/year) |
|---|---|---|---|
| JP 2018-500326 A | 11 January 2018 | US 2018/0000749 A1<br>claims, paragraphs [0172]-[0180]<br>WO 2016/100579 A1<br>EP 3233786 A1<br>KR 10-2017-0096139 A<br>CN 107531616 A | |
| WO 2010/087447 A1 | 05 August 2010 | US 2011/0294901 A1<br>paragraphs [0095]-[0100]<br>EP 2392354 A1<br>CA 2751236 A<br>AU 2010208880 A<br>SG 173158 A<br>MX 2011007754 A<br>CN 102365098 A<br>RU 2011135958 A<br>BR PI1007557 A<br>KR 10-2011-0117145 A | |
| JP 2007-332097 A | 27 December 2007 | (Family: none) | |
| JP 2006-306766 A | 09 November 2006 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009061744 A
- WO 2016100579 A
- WO 2021167095 A
- US 3947473 A
- US 4003919 A
- US 4026907 A


### Non-patent literature cited in the description


- Japanese Pharmacopoeia